# EUROPEAN PATENT APPLICATION

(11) **EP 3 488 941 A1**
(43) Date of publication of application: **29.05.2019**
(21) Application number: 18203982.6
(22) Date of filing: 22.03.2012
(51) Int. Cl.: B09C 1/10, C12Q 1/44, G01N 27/327, H01L 51/00, C07K 5/09

(54) **AROMATIC-CATIONIC PEPTIDES AND USES OF SAME**

(30) Priority: 11.04.2011 US 201161474189 P; 28.04.2011 US 201161480267 P; 24.03.2011 US 201161467288 P
(62) Divisional of application: 16165849.7
(71) Applicant: Cornell University, Ithaca, NY 14850 (US); Stealth Peptides International, Inc., 98000 Monaco (MC)
(72) Inventor: SZETO, Hazel H., New York, 10128 (US); BIRK, Alexander V., New York, 11426 (US); WILSON, D. Travis, Massachusetts, 02461 (US)
(74) Representative: J A Kemp

(57) **Abstract**

The present disclosure provides aromatic-cationic peptide compositions and methods of using the same. The methods comprise use of the peptides in electron transport and electrical conductance.

## Description

### TECHNICAL FIELD

The present technology relates generally to aromatic-cationic peptide compositions and methods of use in electron transport and electrical conductance.

### SUMMARY

In one aspect, the present technology provides an aromatic-cationic peptide or a pharmaceutically acceptable salt thereof such as acetate salt or trifluoroacetate salt. In some embodiments, the peptide comprises
1. at least one net positive charge;
2. a minimum of three amino acids;
3. a maximum of about twenty amino acids;
4. a relationship between the minimum number of net positive charges (pₘ) and the total number of amino acid residues (r) wherein 3pₘ is the largest number that is less than or equal to r + 1; and
5. a relationship between the minimum number of aromatic groups (a) and the total number of net positive charges (pₜ) wherein 2a is the largest number that is less than or equal to pₜ + 1, except that when a is 1, pₜ may also be 1.

In some embodiments, the peptide comprises the amino acid sequence Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt -Lys-Phe-NH₂ (SS-31). In some embodiments, the peptide is comprises one or more of:
D-Arg-Dmt-Lys-Trp-NH₂;
D-Arg-Trp-Lys-Trp-NH₂;
D-Arg-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]-Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂; and
H-D-Arg-Dmt-Ψ[CH₂-NH]Lys-Ψ[CH₂-NH]Phe-NH₂;
Lys-D-Arg-Tyr-NH₂,
Tyr-D-Arg-Phe-Lys-NH2
2',6'-Dmt-D-Arg-Phe-Lys-NH2
Phe-D-Arg-Phe-Lys-NH2
Phe-D-Arg-Dmt-Lys-NH2
D-Arg-2'6'Dmt-Lys-Phe-NH2
H-Phe-D-Arg-Phe-Lys-Cys-NH2
Lys-D-Arg-Tyr-NH2
D-Tyr-Trp-Lys-NH₂,
Trp-D-Lys-Tyr-Arg-NH₂,
Tyr-His-D-Gly-Met,
Tyr-D-Arg-Phe-Lys-Glu-NH₂,
Met-Tyr-D-Lys-Phe-Arg,
D-His-Glu-Lys-Tyr-D-Phe-Arg,
Lys-D-Gln-Tyr-Arg-D-Phe-Trp-NH₂,
Phe-D-Arg-Lys-Trp-Tyr-D-Arg-His,
Gly-D-Phe-Lys-Tyr-His-D-Arg-Tyr-NH₂,
Val-D-Lys-His-Tyr-D-Phe-Ser-Tyr-Arg-NH₂,
Trp-Lys-Phe-D-Asp-Arg-Tyr-D-His-Lys,
Lys-Trp-D-Tyr-Arg-Asn-Phe-Tyr-D-His-NH₂,
Thr-Gly-Tyr-Arg-D-His-Phe-Trp-D-His-Lys,
Asp-D-Trp-Lys-Tyr-D-His-Phe-Arg-D-Gly-Lys-NH₂,
D-His-Lys-Tyr-D-Phe-Glu-D-Asp-D-His-D-Lys-Arg-Trp-NH₂,
Ala-D-Phe-D-Arg-Tyr-Lys-D-Trp-His-D-Tyr-Gly-Phe,
Tyr-D-His-Phe-D-Arg-Asp-Lys-D-Arg-His-Trp-D-His-Phe,
Phe-Phe-D-Tyr-Arg-Glu-Asp-D-Lys-Arg-D-Arg-His-Phe-NH₂,
Phe-Tyr-Lys-D-Arg-Trp-His-D-Lys-D-Lys-Glu-Arg-D-Tyr-Thr,
Tyr-Asp-D-Lys-Tyr-Phe-D-Lys-D-Arg-Phe-Pro-D-Tyr-His-Lys,
Glu-Arg-D-Lys-Tyr-D-Val-Phe-D-His-Trp-Arg-D-Gly-Tyr-Arg-D-Met-NH₂,
Arg-D-Leu-D-Tyr-Phe-Lys-Glu-D-Lys-Arg-D-Trp-Lys-D-Phe-Tyr-D-Arg-Gly,
D-Glu-Asp-Lys-D-Arg-D-His-Phe-Phe-D-Val-Tyr-Arg-Tyr-D-Tyr-Arg-His-Phe-NH₂,
Asp-Arg-D-Phe-Cys-Phe-D-Arg-D-Lys-Tyr-Arg-D-Tyr-Trp-D-His-Tyr-D-Phe-Lys-Phe,
His-Tyr-D-Arg-Trp-Lys-Phe-D-Asp-Ala-Arg-Cys-D- Tyr-His-Phe-D-Lys-Tyr-His-Ser-NH₂,
Gly-Ala-Lys-Phe-D-Lys-Glu-Arg-Tyr-His-D-Arg-D-Arg-Asp-Tyr-Trp-D-His-Trp-His-D-Lys-Asp, and
Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂.

In some embodiments, "Dmt" refers to 2',6'-dimethyltyrosine (2',6'-Dmt). In some embodiments, "Dmt" refers to 3',5'-dimethyltyrosine (3',5'Dmt).

In one embodiment, the peptide is defined by formula I: 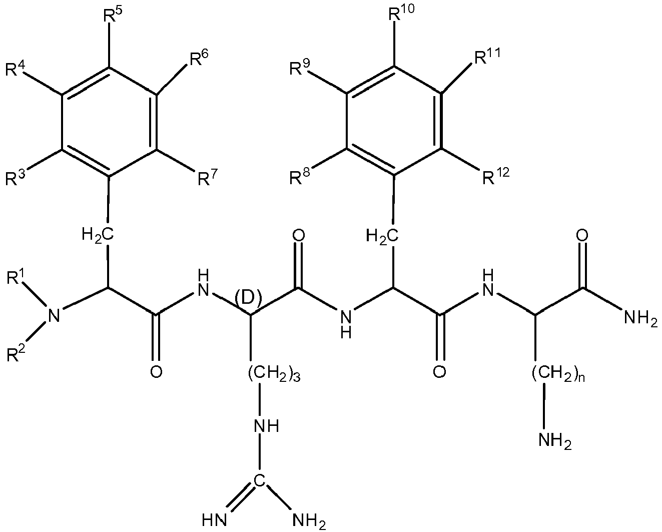
wherein R¹ and R² are each independently selected from
   (i) hydrogen;
   (ii) linear or branched C₁-C₆ alkyl;
   (iii) 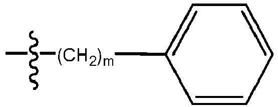 where m = 1-3;
   (iv) 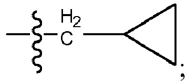
   (v) 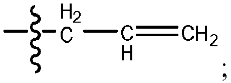
R³, R⁴, R⁵, R⁶, R⁷, R⁸, R⁹, R¹⁰, R¹¹ and R¹² are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and
n is an integer from 1 to 5.

In a particular embodiment, R¹, R², R³, R⁴, R⁵, R⁶, R⁷, R⁸, R⁹, R¹⁰, R¹¹, and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

In one embodiment, the peptide is defined by formula II: 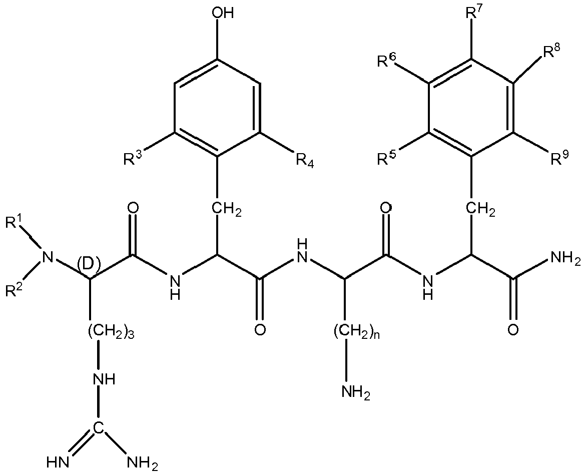
wherein R¹ and R² are each independently selected from
   (i) hydrogen;
   (ii) linear or branched C₁-C₆ alkyl;
   (iii) 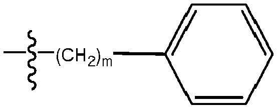 where m = 1-3;
   (iv) 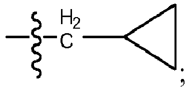
   (v) 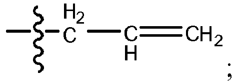
R³ and R⁴ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo;
R⁵, R⁶, R⁷, R⁸, and R⁹ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and
n is an integer from 1 to 5.

In a particular embodiment, R¹ and R² are hydrogen; R³ and R⁴ are methyl; R⁵, R⁶, R⁷, R⁸, and R⁹ are all hydrogen; and n is 4.

In one embodiment, the aromatic-cationic peptides have a core structural motif of alternating aromatic and cationic amino acids. Fr example, the peptide may be a tetrapeptide defined by any of formulas III to VI set forth below:

Aromatic - Cationic - Aromatic - Cationic (Formula III)

Cationic - Aromatic - Cationic - Aromatic (Formula IV)

Aromatic - Aromatic - Cationic - Cationic (Formula V)

Cationic - Cationic - Aromatic - Aromatic (Formula VI)

wherein, Aromatic is a residue selected from the group consisting of: Phe (F), Tyr (Y), Trp (W), and Cyclohexylalanine (Cha); and Cationic is a residue selected from the group consisting of: Arg (R), Lys (K), Norleucine (Nle), and 2-amino-heptanoic acid (Ahe).

In some embodiments, the aromatic-cationic peptides described herein comprise all levorotatory (L) amino acids.

In some aspects, the present disclosures provides methods relating to cytochrome c. In some embodiments, the method relates to increasing cytochrome c reduction in a sample containing cytochrome c, comprising contacting the sample with an effective amount of an aromatic-cationic peptide or a salt thereof, such as acetate or trifluoroacetate salt. Additionally or alternatively, in some embodiments, the method relates to enhancing electron diffusion through cytochrome c in a sample containing cytochrome c, comprising contacting the sample with an effective amount of an aromatic-cationic peptide. Additionally or alternatively, in some embodiments, the method relates to enhancing electron capacity in cytochrome c in a sample containing cytochrome c, comprising contacting the sample with an effective amount of an aromatic-cationic peptide. Additionally or alternatively, in some embodiments, the method relates to inducing a novel π-π interaction around cytochrome c in a sample containing cytochrome c, comprising contacting the sample with an effective amount of an aromatic-cationic peptide. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some embodiments, the sample containing cytochrome c doped with an aromatic-cationic peptide of the invention comprises a component of a sensor, such as a photocell or luminescent sensor; a conductor; a switch, such as a transistor; a light emitting element, such as a light emitting diode; a charge storage or accumulation device, such as a photovoltaic device; a diode; an integrated circuit; a solid-state device; or any other organic electronic devices. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some embodiments, cytochrome c is present in a sample in purified, isolated and/or concentrated form. In some embodiments, cytochrome c is present in a sample in a natural form. For example, in some embodiments, cytochrome c is present in one or more mitochrondria. In some embodiments, the mitochondria are isolated. In other embodiments, the mitochondria are present in a cell or in a cellular preparation. In some embodiments, the cytochrome c is doped with an aromatic-cationic peptide or a salt thereof, such as acetate or trifluoroacetate salt. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some aspects, the present disclosure provides a methods relating to mitochondrial respiration. In some embodiments, the method relates to increasing mitochondrial O₂ consumption, increasing ATP synthesis in a sample, and/or enhancing respiration in cytochrome c-depleted mitoplasts. In some embodiments, a sample containing mitochrodria, and/or cytochrome depleted mitoplasts is contacted with an effective amount of an aromatic-cationic peptide, or a salt thereof. In some embodiments, the mitochondria are present in a sample in purified, isolated and/or concentrated form. In some embodiments, the mitochondria are present in a sample in a natural form. For example, in some embodiments, the mitochondria are present in a cell or in a cellular preparation. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some aspects, a sensor is provided. In some embodiments, the sensor includes cytochrome c doped with a level of an aromatic-cationic peptide disclosed herein, or a salt thereof, such acetate or trifluoroacetate salt. In some embodiments, the sensor includes a meter to measure a change in a property of the cytochrome c induced by a change in the level of the aromatic-cationic peptide. In some embodiments, the level of the aromatic-cationic peptide changes in response to variation in at least one of a temperature of the cytochrome c and a pH of the cytochrome c. In some embodiments, the property is conductivity and the meter includes an anode and a cathode in electrical communication with the cytochrome c. In some embodiments, the property is photoluminescence and the meter includes a photodetector to measure a change in at least one of an intensity of light emitted by the cytochrome c doped with a level of an aromatic-cationic peptide of the invention and wavelength of light emitted by the peptide-doped cytochrome c. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some aspects, a method of sensing is provided. In some embodiments, the method comprises measuring a change in a property of cytochrome c doped with a level of an aromatic-cationic peptide or a salt thereof, such as acetate or trifluoroacetate salt. In some embodiments, the change measured is induced by a change in the level of the aromatic-cationic peptide. In some embodiments, the level of the aromatic-cationic peptide changes in response to variation in at least one of a temperature of the cytochrome c and a pH of the cytochrome c. In some embodiments, the property is at least one of conductivity, photoluminescent intensity, and photoluminescent wavelength. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some aspects a switch is provided. In some embodiments, the switch comprises cytochrome c and a source of an aromatic-cationic peptide. In some embodiments, the aromatic-cationic peptide or a salt thereof, such as acetate or trifluoroacetate salt is in communication with the cytochrome c. In some embodiments, an actuator is provided to control an amount of the aromatic-cationic peptide in communication with the cytochrome c. In some embodiments, the actuator controls at least one of a temperature of the cytochrome c and a pH of the cytochrome c. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some aspects, a method of switching is provided. In some embodiments, the method comprises changing a level of an aromatic-cationic peptide or a salt thereof, such as acetate or trifluoroacetate salt in communication with cytochrome c. In some embodiments, changing a level of an aromatic-cationic peptide includes varying at least one of a temperature of the cyt c and a pH of the cyt c. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂.

In some aspects, a light-emitting element is provided. In some embodiments, the light-emitting element comprises cytochrome c doped with an effective amount of an aromatic-cationic peptide, such as D-Arg-2',6'-Dmt-Lys-Phe-NH₂, and/or Phe-D-Arg-Phe-Lys-NH₂ or a salt thereof, such as acetate or trifluoroacetate salt and a source to stimulate emission of light from the cytochrome c.

In some aspects, a method of emitting light is provided. In some embodiments, the method comprising stimulating cytochrome c doped with an effective amount of an aromatic-cationic peptide or a salt thereof, such as acetate or trifluoroacetate salt, such as D-Arg-2',6'-Dmt-Lys-Phe-NH₂ and/or Phe-D-Arg-Phe-Lys-NH₂.

In some aspects, the present disclosure provides methods and compositions for cytochrome c biosensors. In some embodiments, the cytochrome c biosensor includes one or more of the aromatic-cationic peptides or a salt thereof, such as acetate or trifluoroacetate salt disclosed herein. In some embodiments, peptide-doped cytochrome c serves as a mediator between a redox-active enzyme and an electrode within the biosensor. In some embodiments, peptide-doped cytochrome c is immobilized directly on the electrode of the biosensor. In some embodiments, the peptide is linked to cytochrome c within the biosensor. In some embodiments, the peptide is not linked to cytochrome c. In some embodiments, the peptide and/or cytochrome c are immobilized on a surface within the biosensor. In some embodiments, the peptide and/or cytochrome c are freely diffusible within the biosensor. In some embodiments, the biosensor includes the peptide D-Arg-2',6'-Dmt-Lys-Phe-NH₂. Additionally or alternatively, in some embodiments, the biosensor includes the aromatic-cationic peptide Phe-D-Arg-Phe-Lys-NH₂.

In some aspects, the present disclosure provides compositions for the bioremediation of environmental contaminants. In some embodiments, the composition comprises recombinant bacteria expressing one or more aromatic-cationic peptides or a salt thereof, such as acetate or trifluoroacetate salt. In some embodiments, the recombinant bacteria comprise a nucleic acid encoding the one or more aromatic-cationic peptides. In some embodiments, the nucleic acid is expressed under the control of an inducible promoter. In some embodiments, the nucleic acid is expressed under the control of a constitutive promoter. In some embodiments, the nucleic acid comprises a plasmid DNA. In some embodiments, the nucleic acid comprises a genomic insert. In some embodiments, recombinant bacteria are derived from bacterial species listed in Table 7.

In some aspects, the present disclosure provides methods for the bioremediation of environmental contaminants. In some embodiments, the methods comprise contacting a material containing an environmental contaminant with a bioremedial composition comprising recombinant bacteria expressing one or more aromatic-cationic peptides. In some embodiments, the methods disclosed herein comprise methods for dissimilatory metal reduction. In some embodiments, the metal comprises Sc, Ti, V, Cr, Mn, Fe, Co, Ni, Cu, Zn, Y, Zr, Nb, Mo, Tc, Ru, Pd, Ag, Cd, Hf, Ta, W, Re, Os, Ir, Pt, Au, Hg, Rf, Db, Sg, Bh, Hs, Cn, Al, Ga, In, Sn, Ti, Pb, or Bi. In some embodiments, the methods disclosed herein comprise methods for dissimilatory reduction of a non-metal. In some embodiments, the non-metal comprises sulfate. In some embodiments, the methods disclosed herein comprise methods for dissimilatory reduction of perchlorate. In some embodiments, the perchlorate comprises NH₄ClO₄, CsClO₄, LiClO₄, Mg(ClO₄)₂, HClO₄, KClO₄, RbClO₄, AgClO₄, or NaClO₄. In some embodiments, the methods disclosed herein comprise methods for dissimilatory nitrate reduction. In some embodiments, the nitrate comprises HNO₃, LiNO₃, NaNO₃, KNO₃, RbNO₃, CsNO₃, Be(NO₃)₂, Mg(NO₃)₂, Ca(NO₃)₂, Sr(NO₃)₂, Ba(NO₃)₂, Sc(NO₃)₃, Cr(NO₃)₃, Mn(NO₃)₂, Fe(NO₃)₃, CO(N0₃)₂, Ni(NO₃)₂, Cu(NO₃)₂, Zn(NO₃)₂, Pd(NO₃)₂, Cd(NO₃)₂, Hg(NO₃)₂, Pb(NO₃)₂, or Al(NO₃)₃. In some embodiments, the methods disclosed herein comprise methods for dissimilatory reduction of a radionuclide. In some embodiments, the radionuclide comprises an actinide. In some embodiments, the radionuclide comprises uranium. In some embodiments, the methods disclosed herein comprise methods for dissimilatory reduction of methyl-tert-butyl-ether (MTBE), vinyl chloride, or dichloroethylene.

In some embodiments, the bioremediation methods described herein are performed *in situ.* In some embodiments, the bioremediation methods described herein are performed *ex situ.*

In some embodiments, the bioremediation methods described herein comprise contacting a contaminant with recombinant bacteria comprising a nucleic acid encoding one or more aromatic-cationic peptides. In some embodiments, the nucleic acid is expressed under the control of an inducible promoter. In some embodiments, the nucleic acid is expressed under the control of a constitutive promoter. In some embodiments, the nucleic acid comprises a plasmid DNA. In some embodiments, the nucleic acid comprises a genomic insert. In some embodiments, the recombinant bacteria are derived from bacterial species listed in Table 7.

In some embodiments of the bioremediation methods and compositions disclosed herein, the aromatic-cationic peptide comprises D-Arg-2',6'-Dmt-Lys-Phe-NH₂..

### BRIEF DESCRIPTION OF THE FIGURES

FIG. 1A and B are charts showing that the peptide D-Arg-2',6'-Dmt-Lys-Phe-NH₂ (SS-31) increases the rate of cytochrome c reduction.
FIG. 2A is a chart showing that the peptide D-Arg-2',6'-Dmt-Lys-Phe-NH₂ (SS-31) enhances electron diffusion through cytochrome c. FIG 2B is a graph showing a cyclic voltammogram of the cytochrome c in solution with increasing SS-31 doses (20 mM Tris-borate-EDTA (TBE) buffer pH 7 at 100 mV/s.
FIG. 3A and 3B are charts showing that the peptide D-Arg-2',6'-Dmt-Lys-Phe-NH₂ (SS-31) enhances electron capacity in cytochrome c.
FIG. 4 is a chart showing that the peptide D-Arg-2',6'-Dmt-Lys-Phe-NH₂ (SS-31) induces novel π-π interactions around cytochrome c heme.
FIG. 5A and B are charts showing that the peptide D-Arg-2',6'-Dmt-Lys-Phe-NH₂ (SS-31) increases O₂ consumption in isolated mitochondria.
FIG. 6 is a chart showing that the peptide D-Arg-2',6'-Dmt-Lys-Phe-NH₂ (SS-31) increases ATP synthesis in isolated mitochondria.
FIG. 7 is a chart showing that the peptide D-Arg-2',6'-Dmt-Lys-Phe-NH₂ (SS-31) enhances respiration in cytochrome c-depleted mitoplasts.
FIG. 8 is a diagram of a peptide-doped cytochrome c sensor.
FIG. 9 is a diagram of an alternative peptide-doped cytochrome c sensor.
FIG. 10 is a diagram of a peptide-doped cytochrome c switch.
FIG. 11 is a diagram of electron flow in a biosensor in which peptide-doped cytochrome c serves as a mediator in electron flow to an electrode.
FIG. 12 is a diagram of electron flow in a biosensor in which peptide-doped cytochrome c is immobilized on the electrode.
FIG. 13 is a chart showing that the peptides D-Arg-2',6'-Dmt-Lys-Phe-NH₂ (SS-31) and Phe-D-Arg-Phe-Lys-NH₂ (SS-20) facilitate cytochrome c reduction.
FIG. 14 is a chart showing that the peptides D-Arg-2',6'-Dmt-Lys-Phe-NH₂ (SS-31) and Phe-D-Arg-Phe-Lys-NH₂ (SS-20) promote electron flux, as measured by O₂ consumption in isolated rat kidney mitochondria.
FIG. 15 is a chart showing that the peptides D-Arg-2',6'-Dmt-Lys-Phe-NH₂ (SS-31) and Phe-D-Arg-Phe-Lys-NH₂ (SS-20) increase the rate of ATP production in isolated mitochondria.
FIG. 16 is a block diagram of an organic light-emitting transistor.
FIG. 17 is a block diagram of an organic light-emitting diode.
FIG. 18 is a block diagram of a dispersed heterojunction organic photovoltaic cell.
FIG. 19A illustrates electron-hole pair generation with a highly folded heterojunction organic photovoltaic cell. FIG. 19B illustrates electron-hole pair generation with a controlled-growth heterojunction organic photovoltaic cell made.
FIG. 20A and B illustrate techniques for depositing thin films of organic material during manufacture of organic electronic devices, including, but not limited to, organic light-emitting transistors, organic light-emitting diodes, and organic photovoltaic cells

### DETAILED DESCRIPTION

It is to be appreciated that certain aspects, modes, embodiments, variations and features of the invention are described below in various levels of detail in order to provide a substantial understanding of the present invention. The definitions of certain terms as used in this specification are provided below. Unless defined otherwise, all technical and scientific terms used herein generally have the same meaning as commonly understood by one of ordinary skill in the art to which this invention belongs.

In practicing the present disclosure, many conventional techniques of cell biology, molecular biology, protein biochemistry, immunology, and bacteriology are used. These techniques are well-known in the art and are provided in any number of available publications, including Current Protocols in Molecular Biology, Vols. I-III, Ausubel, Ed. (1997); Sambrook et al., Molecular Cloning: A Laboratory Manual, Second Ed. (Cold Spring Harbor Laboratory Press, Cold Spring Harbor, NY, 1989).

As used in this specification and the appended claims, the singular forms "a", "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally-occurring amino acid, *i.e*., an α-carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect. In the context of therapeutic or prophylactic applications, the amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds. In some embodiments the term "effective amount" refers to a quantity sufficient to achieve a desired electronic or conductance effect, *e.g*., to facilitate or enhance electron transfer..

As used herein, "exogenous nucleic acid" refers to nucleic acid (e.g., DNA, RNA) that is not naturally present within a host cell but is introduced from an outside source. As used herein, exogenous nucleic acid refers to nucleic acid that has not integrated in to the genome of the host cell but remains separate, such as a bacterial plasmid nucleic acid. As used herein, "bacterial plasmid" refers to a circular DNA of bacterial origin which serves as a carrier of a sequence of interest and a means for expressing that sequence in a bacterial host cell.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide or an isolated cytochrome c protein would be free of materials that would interfere with diagnostic or therapeutic uses of the agent or would interfere with conductance, or electric properties of the peptide. Such interfering materials may include enzymes, hormones and other proteinaceous and nonproteinaceous solutes.

As used herein, "inducible promoter" refers to a promoter that is influenced by certain conditions, such as temperature or the presence of specific molecules, and promotes the expression of operably linked nucleic acid sequences of interest only when those conditions are met.

As used herein, "constitutive promoter" refers to a promoter that facilitates expression of operably linked nucleic acid sequences of interest under all or most environmental conditions.

As used herein, the terms "polypeptide", "peptide", and "protein" are used interchangeably herein to mean a polymer comprising two or more amino acids joined to each other by peptide bonds or modified peptide bonds, *i.e*., peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, "recombinant bacteria" refers to bacteria that have been engineered to carry and /or express one or more exogenous nucleic acid (e.g., DNA) sequences.

As used herein, the terms "treating" or "treatment" or "alleviation" refers to both therapeutic treatment and prophylactic or preventative measures, wherein the object is to prevent or slow down (lessen) the targeted pathologic condition or disorder. It is also to be appreciated that the various modes of treatment or prevention of medical conditions as described are intended to mean "substantial", which includes total but also less than total treatment or prevention, and wherein some biologically or medically relevant result is achieved.

As used herein, "prevention" or "preventing" of a disorder or condition refers to a compound that reduces the occurrence of the disorder or condition in the treated sample relative to an untreated control sample, or delays the onset or reduces the severity of one or more symptoms of the disorder or condition relative to the untreated control sample.

### Aromatic-cationic peptides

The present technology relates to the use of aromatic-cationic peptides. In some embodiments, the peptides are useful in aspects related to conductance.

The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, about nine, or about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e*., alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid includes hydroxyproline (Hyp).

The peptides optionally contain one or more non-naturally occurring amino acids. Optimally, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy (*i.e.,* alkoxy), amino, C₁-C₄ alkylamino and C₁-C₄ dialkylamino (*e.g*., methylamino, dimethylamino), nitro, hydroxyl, halo (*i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, e.g. methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are suitably resistant or insensitive, to common proteases. Examples of non-naturally occurring amino acids that are resistant or insensitive to proteases include the dextrorotatory (D-) form of any of the above-mentioned naturally occurring L-amino acids, as well as L-and/or D-non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, less than four, less than three, or less than two contiguous L-amino acids recognized by common proteases, irrespective of whether the amino acids are naturally or non-naturally occurring. In one embodiment, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

"Net charge" as used herein refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid (*i.e.,* Glu) and four positively charged amino acids (*i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ≤p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| (**pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

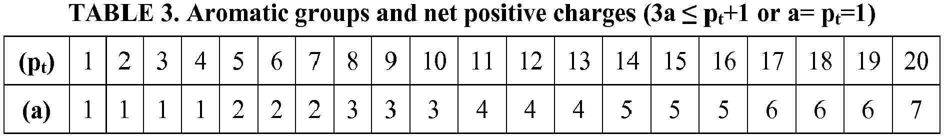

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

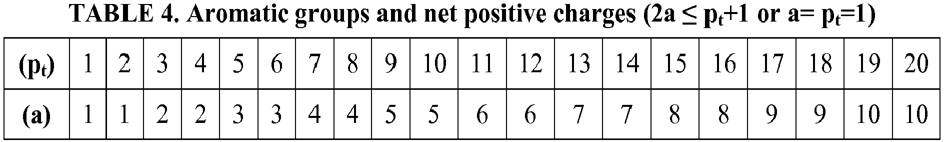

In another embodiment, the number of aromatic groups (a) and the total number of net positive charges (pₜ) are equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid. In a particular embodiment, the aromatic-cationic peptide is a tripeptide having two net positive charges and two aromatic amino acids.

In one embodiment, the aromatic-cationic peptide has
1. at least one net positive charge;
2. a minimum of three amino acids;
3. a maximum of about twenty amino acids;
4. a relationship between the minimum number of net positive charges (pₘ) and the total number of amino acid residues (r) wherein 3pₘ is the largest number that is less than or equal to r + 1; and
5. a relationship between the minimum number of aromatic groups (a) and the total number of net positive charges (pₜ) wherein 2a is the largest number that is less than or equal to pₜ + 1, except that when a is 1, pₜ may also be 1.

In another embodiment, the invention provides a method for reducing the number of mitochondria undergoing a mitochondrial permeability transition (MPT), or preventing mitochondrial permeability transitioning in a removed organ of a mammal. The method comprises administering to the removed organ an effective amount of an aromatic-cationic peptide having:
at least one net positive charge;
a minimum of three amino acids;
a maximum of about twenty amino acids;
a relationship between the minimum number of net positive charges (pₘ) and the total number of amino acid residues (r) wherein 3pₘ is the largest number that is less than or equal to r + 1; and
a relationship between the minimum number of aromatic groups (a) and the total number of net positive charges (pₜ) wherein 2a is the largest number that is less than or equal to pₜ + 1, except that when a is 1, pₜ may also be 1.

In yet another embodiment, the invention provides a method of reducing the number of mitochondria undergoing mitochondrial permeability transition (MPT), or preventing mitochondria permeability transitioning in a mammal in need thereof. The method comprises administering to the mammal an effective amount of an aromatic-cationic peptide having:
at least one net positive charge;
a minimum of three amino acids;
a maximum of about twenty amino acids;
a relationship between the minimum number of net positive charges (pₘ) and the total number of amino acid residues (r) wherein 3 pₘ is the largest number that is less than or equal to r + 1; and
a relationship between the minimum number of aromatic groups (a) and the total number of net positive charges (pₜ) wherein 3a is the largest number that is less than or equal to pₜ + 1, except that when a is 1, pₜ may also be 1.

Aromatic-cationic peptides include, but are not limited to, the following illustrative peptides:
H-Phe-D-Arg Phe-Lys-Cys-NH₂
D-Arg-Dmt-Lys-Trp-NH₂;
D-Arg-Trp-Lys-Trp-NH₂;
D-Arg-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt-(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂; and
H-D-Arg-Dmt-Ψ[CH₂-NH]Lys-Ψ[CH₂-NH]Phe-NH₂,
Tyr-D-Arg-Phe-Lys-NH2
2',6'-Dmt-D-Arg-Phe-Lys-NH2
Phe-D-Arg-Phe-Lys-NH2
Phe-D-Arg-Dmt-Lys-NH2
D-Arg-2',6'-Dmt-Lys-Phe-NH2
H-Phe-D-Arg-Phe-Lys-Cys-NH2
Lys-D-Arg-Tyr-NH₂,
D-Tyr-Trp-Lys-NH₂,
Trp-D-Lys-Tyr-Arg-NH₂,
Tyr-His-D-Gly-Met,
Tyr-D-Arg-Phe-Lys-Glu-NH₂,
Met-Tyr-D-Lys-Phe-Arg,
D-His-Glu-Lys-Tyr-D-Phe-Arg,
Lys-D-Gln-Tyr-Arg-D-Phe-Trp-NH₂,
Phe-D-Arg-Lys-Trp-Tyr-D-Arg-His,
Gly-D-Phe-Lys-Tyr-His-D-Arg-Tyr-NH₂,
Val-D-Lys-His-Tyr-D-Phe-Ser-Tyr-Arg-NH₂,
Trp-Lys-Phe-D-Asp-Arg-Tyr-D-His-Lys,
Lys-Trp-D-Tyr-Arg-Asn-Phe-Tyr-D-His-NH₂,
Thr-Gly-Tyr-Arg-D-His-Phe-Trp-D-His-Lys,
Asp-D-Trp-Lys-Tyr-D-His-Phe-Arg-D-Gly-Lys-NH₂,
D-His-Lys-Tyr-D-Phe-Glu-D-Asp-D-His-D-Lys-Arg-Trp-NH₂,
Ala-D-Phe-D-Arg-Tyr-Lys-D-Trp-His-D-Tyr-Gly-Phe,
Tyr-D-His-Phe-D-Arg-Asp-Lys-D-Arg-His-Trp-D-His-Phe,
Phe-Phe-D-Tyr-Arg-Glu-Asp-D-Lys-Arg-D-Arg-His-Phe-NH₂,
Phe-Tyr-Lys-D-Arg-Trp-His-D-Lys-D-Lys-Glu-Arg-D-Tyr-Thr,
Tyr-Asp-D-Lys-Tyr-Phe-D-Lys-D-Arg-Phe-Pro-D-Tyr-His-Lys,
Glu-Arg-D-Lys-Tyr-D-Val-Phe-D-His-Trp-Arg-D-Gly-Tyr-Arg-D-Met-NH₂,
Arg-D-Leu-D-Tyr-Phe-Lys-Glu-D-Lys-Arg-D-Trp-Lys-D-Phe-Tyr-D-Arg-Gly,
D-Glu-Asp-Lys-D-Arg-D-His-Phe-Phe-D-Val-Tyr-Arg-Tyr-D-Tyr-Arg-His-Phe-NH₂,
Asp-Arg-D-Phe-Cys-Phe-D-Arg-D-Lys-Tyr-Arg-D-Tyr-Trp-D-His-Tyr-D-Phe-Lys-Phe,
His-Tyr-D-Arg-Trp-Lys-Phe-D-Asp-Ala-Arg-Cys-D- Tyr-His-Phe-D-Lys-Tyr-His-Ser-NH₂,
Gly-Ala-Lys-Phe-D-Lys-Glu-Arg-Tyr-His-D-Arg-D-Arg-Asp-Tyr-Trp-D-His-Trp-His-D-Lys-Asp, and
Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂.

In some embodiments, peptides useful in the methods of the present invention are those peptides which have a tyrosine residue or a tyrosine derivative. In some embodiments, derivatives of tyrosine include 2'-methyltyrosine (Mmt); 2',6'-dimethyltyrosine (2' 6'-Dmt); 3',5'-dimethyltyrosine (3',5'-Dmt); N,2',6'-trimethyltyrosine (Tmt); and 2'-hydroxy-6'-methyltryosine (Hmt).

In one embodiment, the peptide has the formula Tyr-D-Arg-Phe-Lys-NH₂ (referred to herein as SS-01). SS-01 has a net positive charge of three, contributed by the amino acids tyrosine, arginine, and lysine and has two aromatic groups contributed by the amino acids phenylalanine and tyrosine. The tyrosine of SS-01 can be a modified derivative of tyrosine such as in 2',6'-dimethyltyrosine to produce the compound having the formula 2',6'-Dmt-D-Arg-Phe-Lys-NH₂ (referred to herein as SS-02).

In a suitable embodiment, the amino acid residue at the N-terminus is arginine. An example of such a peptide is D-Arg-2',6'-Dmt-Lys-Phe-NH₂ (referred to herein as SS-31).

In another embodiment, the amino acid at the N-terminus is phenylalanine or its derivative. In some embodiments, derivatives of phenylalanine include 2'-methylphenylalanine (Mmp), 2',6'-dimethylphenylalanine (Dmp), N,2',6'-trimethylphenylalanine (Tmp), and 2'-hydroxy-6'-methylphenylalanine (Hmp). An example of such a peptide is Phe-D-Arg-Phe-Lys-NH₂ (referred to herein as SS-20). In one embodiment, the amino acid sequence of SS-02 is rearranged such that 2',6'-Dmt is not at the N-terminus. An example of such an aromatic-cationic peptide has the formula D-Arg-2',6'-Dmt-Lys-Phe-NH₂ (SS-31).

In yet another embodiment, the aromatic-cationic peptide has the formula Phe-D-Arg-2',6'-Dmt-Lys-NH₂ (referred to herein as SS-30). Alternatively, the N-terminal phenylalanine can be a derivative of phenylalanine such as 2',6'-dimethylphenylalanine (2'6'Dmp). In some embodiments, the peptide has a formula containing 2',6'-dimethylphenylalanine at amino acid position one and has the formula 2',6'-Dmp-D-Arg-2',6'-Dmt-Lys-NH₂. In some embodiments, the peptide has a formula 2',6'-Dmp-D-Arg-Phe-Lys-NH₂.

The peptides mentioned herein and their derivatives can further include functional analogs. A peptide is considered a functional analog if the analog has the same function as the stated peptide. The analog may, for example, be a substitution variant of a peptide, wherein one or more amino acids are substituted by another amino acid. Suitable substitution variants of the peptides include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide. Non-limiting examples of analogs useful in the practice of the present invention include, but are not limited to, the aromatic-cationic peptides shown in Table 5.

**TABLE 5. Examples of Peptide Analogs**

| **Amino Acid Position 1** | **Amino Acid Position 2** | **Amino Acid Position 3** | **Amino Acid Position 4** | **Amino Acid Position 5** | **Amino Acid Position 6** | **Amino Acid Position 7** | **C- Terminal Modification** |
|---|---|---|---|---|---|---|---|
| D-Arg | Dmt | Lys | Phe | | | | NH₂ |
| D-Arg | Dmt | Phe | Lys | | | | NH₂ |
| D-Arg | Phe | Lys | Dmt | | | | NH₂ |
| D-Arg | Phe | Dmt | Lys | | | | NH₂ |
| D-Arg | Lys | Dmt | Phe | | | | NH₂ |
| D-Arg | Lys | Phe | Dmt | | | | NH₂ |
| D-Arg | Dmt | Lys | Phe | Cys | | | NH₂ |
| D-Arg | Dmt | Lys | Phe | Glu | Cys | Gly | NH₂ |
| D-Arg | Dmt | Lys | Phe | Ser | Cys | | NH₂ |
| D-Arg | Dmt | Lys | Phe | Gly | Cys | | NH₂ |
| Phe | Lys | Dmt | D-Arg | | | | NH₂ |
| Phe | Lys | D-Arg | Dmt | | | | NH₂ |
| Phe | D-Arg | Phe | Lys | | | | NH₂ |
| Phe | D-Arg | Phe | Lys | Cys | | | NH₂ |
| Phe | D-Arg | Phe | Lys | Glu | Cys | Gly | NH₂ |
| Phe | D-Arg | Phe | Lys | Ser | Cys | | NH₂ |
| Phe | D-Arg | Phe | Lys | Gly | Cys | | NH₂ |
| Phe | D-Arg | Dmt | Lys | | | | NH₂ |
| Phe | D-Arg | Dmt | Lys | Cys | | | NH₂ |
| Phe | D-Arg | Dmt | Lys | Glu | Cys | Gly | NH₂ |
| Phe | D-Arg | Dmt | Lys | Ser | Cys | | NH₂ |
| Phe | D-Arg | Dmt | Lys | Gly | Cys | | NH₂ |
| Phe | D-Arg | Lys | Dmt | | | | NH₂ |
| Phe | Dmt | D-Arg | Lys | | | | NH₂ |
| Phe | Dmt | Lys | D-Arg | | | | NH₂ |
| Lys | Phe | D-Arg | Dmt | | | | NH₂ |
| Lys | Phe | Dmt | D-Arg | | | | NH₂ |
| Lys | Dmt | D-Arg | Phe | | | | NH₂ |
| Lys | Dmt | Phe | D-Arg | | | | NH₂ |
| Lys | D-Arg | Phe | Dmt | | | | NH₂ |
| Lys | D-Arg | Dmt | Phe | | | | NH₂ |
| D-Arg | Dmt | D-Arg | Phe | | | | NH₂ |
| D-Arg | Dmt | D-Arg | Dmt | | | | NH₂ |
| D-Arg | Dmt | D-Arg | Tyr | | | | NH₂ |
| D-Arg | Dmt | D-Arg | Trp | | | | NH₂ |
| Trp | D-Arg | Phe | Lys | | | | NH₂ |
| Trp | D-Arg | Tyr | Lys | | | | NH₂ |
| Trp | D-Arg | Trp | Lys | | | | NH₂ |
| Trp | D-Arg | Dmt | Lys | | | | NH₂ |
| D-Arg | Trp | Lys | Phe | | | | NH₂ |
| D-Arg | Trp | Phe | Lys | | | | NH₂ |
| D-Arg | Trp | Lys | Dmt | | | | NH₂ |
| D-Arg | Trp | Dmt | Lys | | | | NH₂ |
| D-Arg | Lys | Trp | Phe | | | | NH₂ |
| D-Arg | Lys | Trp | Dmt | | | | NH₂ |
| Cha | D-Arg | Phe | Lys | | | | NH₂ |
| Ala | D-Arg | Phe | Lys | | | | NH₂ |

| | | | | | | | |
|---|---|---|---|---|---|---|---|
| Cha = cyclohexyl | | | | | | | |

Under certain circumstances, it may be advantageous to use a peptide that also has opioid receptor agonist activity. Examples of analogs useful in the practice of the present invention include, but are not limited to, the aromatic-cationic peptides shown in Table 6.

**TABLE 6. Peptide Analogs with Opioid Receptor Agonist Activity**

| **Amino Acid Position 1** | **Amino Acid Position 2** | **Amino Acid Position 3** | **Amino Acid Position 4** | **Amino Acid Position 5 (if present)** | **C-Terminal Modification** |
|---|---|---|---|---|---|
| Tyr | D-Arg | Phe | Lys | | **NH₂** |
| Tyr | D-Arg | Phe | Orn | | NH₂ |
| Tyr | D-Arg | Phe | Dab | | NH₂ |
| Tyr | D-Arg | Phe | Dap | | NH₂ |
| Tyr | D-Arg | Phe | Lys | Cys | NH₂ |
| 2',6'-Dmt | D-Arg | Phe | Lys | | NH₂ |
| 2',6'-Dmt | D-Arg | Phe | Lys | Cys | NH₂ |
| 2',6'-Dmt | D-Arg | Phe | Lys-NH(CH₂)₂-NH-dns | | NH₂ |
| 2',6'-Dmt | D-Arg | Phe | Lys-NH(CH₂)₂-NH-atn | | NH₂ |
| 2',6'-Dmt | D-Arg | Phe | dnsLys | | NH₂ |
| 2',6'-Dmt | D-Cit | Phe | Lys | | NH₂ |
| 2',6'-Dmt | D-Cit | Phe | Lys | Cys | NH₂ |
| 2',6'-Dmt | D-Cit | Phe | Ahp | | NH₂ |
| 2',6'-Dmt | D-Arg | Phe | Orn | | NH₂ |
| 2',6'-Dmt | D-Arg | Phe | Dab | | NH₂ |
| 2',6'-Dmt | D-Arg | Phe | Dap | | NH₂ |
| 2',6'-Dmt | D-Arg | Phe | Ahp(2-aminoheptanoic acid) | | NH₂ |
| Bio-2',6'-Dmt | D-Arg | Phe | Lys | | NH₂ |
| 3',5-'Dmt | D-Arg | Phe | Lys | | NH₂ |
| 3',5-'Dmt | D-Arg | Phe | Orn | | NH₂ |
| 3',5-'Dmt | D-Arg | Phe | Dab | | NH₂ |
| 3',5-'Dmt | D-Arg | Phe | Dap | | NH₂ |
| Tyr | D-Arg | Tyr | Lys | | NH₂ |
| Tyr | D-Arg | Tyr | Orn | | NH₂ |
| Tyr | D-Arg | Tyr | Dab | | NH₂ |
| Tyr | D-Arg | Tyr | Dap | | NH₂ |
| 2',6'-Dmt | D-Arg | Tyr | Lys | | NH₂ |
| 2',6'-Dmt | D-Arg | Tyr | Orn | | NH₂ |
| 2',6'-Dmt | D-Arg | Tyr | Dab | | NH₂ |
| 2',6'-Dmt | D-Arg | Tyr | Dap | | NH₂ |
| 2',6'-Dmt | D-Arg | 2',6'-Dmt | Lys | | NH₂ |
| 2',6'-Dmt | D-Arg | 2',6'-Dmt | Orn | | NH₂ |
| 2',6'-Dmt | D-Arg | 2',6'-Dmt | Dab | | NH₂ |
| 2',6'-Dmt | D-Arg | 2',6'-Dmt | Dap | | NH₂ |
| 3',5-'Dmt | D-Arg | 3',5-'Dmt | Arg | | NH₂ |
| 3',5-'Dmt | D-Arg | 3',5-'Dmt | Lys | | NH₂ |
| 3',5-'Dmt | D-Arg | 3',5-'Dmt | Orn | | NH₂ |
| 3',5-'Dmt | D-Arg | 3',5-'Dmt | Dab | | NH₂ |
| 2',6'-Dmt | D-Arg | 2',6'-Dmt | Lys | Cys | NH₂ |
| Tyr | D-Lys | Phe | Dap | | NH₂ |
| Tyr | D-Lys | Phe | Arg | | NH₂ |
| Tyr | D-Lys | Phe | Arg | Cys | NH₂ |
| Tyr | D-Lys | Phe | Lys | | NH₂ |
| Tyr | D-Lys | Phe | Orn | | NH₂ |
| 2',6'-Dmt | D-Lys | Phe | Dab | | NH₂ |
| 2',6'-Dmt | D-Lys | Phe | Dap | | NH₂ |
| 2',6'-Dmt | D-Lys | Phe | Arg | | NH₂ |
| 2',6'-Dmt | D-Lys | Phe | Lys | | NH₂ |
| 3',5-'Dmt | D-Lys | Phe | Orn | | NH₂ |
| 3',5-'Dmt | D-Lys | Phe | Dab | | NH₂ |
| 3',5-'Dmt | D-Lys | Phe | Dap | | NH₂ |
| 3',5-'Dmt | D-Lys | Phe | Arg | | NH₂ |
| 3',5-'Dmt | D-Lys | Phe | Arg | Cys | NH₂ |
| Tyr | D-Lys | Tyr | Lys | | NH₂ |
| Tyr | D-Lys | Tyr | Orn | | NH₂ |
| Tyr | D-Lys | Tyr | Dab | | NH₂ |
| Tyr | D-Lys | Tyr | Dap | | NH₂ |
| 2',6'-Dmt | D-Lys | Tyr | Lys | | NH₂ |
| 2',6'-Dmt | D-Lys | Tyr | Orn | | NH₂ |
| 2',6'-Dmt | D-Lys | Tyr | Dab | | NH₂ |
| 2',6'-Dmt | D-Lys | Tyr | Dap | | NH₂ |
| 2',6'-Dmt | D-Lys | 2',6'-Dmt | Lys | | NH₂ |
| 2',6'-Dmt | D-Lys | 2',6'-Dmt | Orn | | NH₂ |
| 2',6'-Dmt | D-Lys | 2',6'-Dmt | Dab | | NH₂ |
| 2',6'-Dmt | D-Lys | 2',6'-Dmt | Dap | | NH₂ |
| 2',6'-Dmt | D-Arg | Phe | dnsDap | | NH₂ |
| 2',6'-Dmt | D-Arg | Phe | atnDap | | NH₂ |
| 3',5-'Dmt | D-Lys | 3',5-'Dmt | Lys | | NH₂ |
| 3',5-'Dmt | D-Lys | 3',5-'Dmt | Orn | | NH₂ |
| 3',5-'Dmt | D-Lys | 3',5-'Dmt | Dab | | NH₂ |
| 3',5-'Dmt | D-Lys | 3',5-'Dmt | Dap | | NH₂ |
| Tyr | D-Lys | Phe | Arg | | NH₂ |
| Tyr | D-Orn | Phe | Arg | | NH₂ |
| Tyr | D-Dab | Phe | Arg | | NH₂ |
| Tyr | D-Dap | Phe | Arg | | NH₂ |
| 2',6'-Dmt | D-Arg | Phe | Arg | | NH₂ |
| 2',6'-Dmt | D-Lys | Phe | Arg | | NH₂ |
| 2',6'-Dmt | D-Orn | Phe | Arg | | NH₂ |
| 2',6'-Dmt | D-Dab | Phe | Arg | | NH₂ |
| 3',5-'Dmt | D-Dap | Phe | Arg | | NH₂ |
| 3',5-'Dmt | D-Arg | Phe | Arg | | NH₂ |
| 3',5-'Dmt | D-Lys | Phe | Arg | | NH₂ |
| 3',5-'Dmt | D-Orn | Phe | Arg | | NH₂ |
| Tyr | D-Lys | Tyr | Arg | | NH₂ |
| Tyr | D-Orn | Tyr | Arg | | NH₂ |
| Tyr | D-Dab | Tyr | Arg | | NH₂ |
| Tyr | D-Dap | Tyr | Arg | | NH₂ |
| 2',6'-Dmt | D-Arg | 2',6'-Dmt | Arg | | NH₂ |
| 2',6'-Dmt | D-Lys | 2',6'-Dmt | Arg | | NH₂ |
| 2',6'-Dmt | D-Orn | 2',6'-Dmt | Arg | | NH₂ |
| 2',6'-Dmt | D-Dab | 2',6'-Dmt | Arg | | NH₂ |
| 3',5-'Dmt | D-Dap | 3',5-'Dmt | Arg | | NH₂ |
| 3',5-'Dmt | D-Arg | 3',5-'Dmt | Arg | | NH₂ |
| 3',5-'Dmt | D-Lys | 3',5-'Dmt | Arg | | NH₂ |
| 3',5-'Dmt | D-Orn | 3',5-'Dmt | Arg | | NH₂ |
| Mmt | D-Arg | Phe | Lys | | NH₂ |
| Mmt | D-Arg | Phe | Orn | | NH₂ |
| Mmt | D-Arg | Phe | Dab | | NH₂ |
| Mmt | D-Arg | Phe | Dap | | NH₂ |
| Tmt | D-Arg | Phe | Lys | | NH₂ |
| Tmt | D-Arg | Phe | Orn | | NH₂ |
| Tmt | D-Arg | Phe | Dab | | NH₂ |
| Tmt | D-Arg | Phe | Dap | | NH₂ |
| Hmt | D-Arg | Phe | Lys | | NH₂ |
| Hmt | D-Arg | Phe | Orn | | NH₂ |
| Hmt | D-Arg | Phe | Dab | | NH₂ |
| Hmt | D-Arg | Phe | Dap | | NH₂ |
| Mmt | D-Lys | Phe | Lys | | NH₂ |
| Mmt | D-Lys | Phe | Orn | | NH₂ |
| Mmt | D-Lys | Phe | Dab | | NH₂ |
| Mmt | D-Lys | Phe | Dap | | NH₂ |
| Mmt | D-Lys | Phe | Arg | | NH₂ |
| Tmt | D-Lys | Phe | Lys | | NH₂ |
| Tmt | D-Lys | Phe | Orn | | NH₂ |
| Tmt | D-Lys | Phe | Dab | | NH₂ |
| Tmt | D-Lys | Phe | Dap | | NH₂ |
| Tmt | D-Lys | Phe | Arg | | NH₂ |
| Hmt | D-Lys | Phe | Lys | | NH₂ |
| Hmt | D-Lys | Phe | Orn | | NH₂ |
| Hmt | D-Lys | Phe | Dab | | NH₂ |
| Hmt | D-Lys | Phe | Dap | | NH₂ |
| Hmt | D-Lys | Phe | Arg | | NH₂ |
| Mmt | D-Lys | Phe | Arg | | NH₂ |
| Mmt | D-Orn | Phe | Arg | | NH₂ |
| Mmt | D-Dab | Phe | Arg | | NH₂ |
| Mmt | D-Dap | Phe | Arg | | NH₂ |
| Mmt | D-Arg | Phe | Arg | | NH₂ |
| Tmt | D-Lys | Phe | Arg | | NH₂ |
| Tmt | D-Orn | Phe | Arg | | NH₂ |
| Tmt | D-Dab | Phe | Arg | | NH₂ |
| Tmt | D-Dap | Phe | Arg | | NH₂ |
| Tmt | D-Arg | Phe | Arg | | NH₂ |
| Hmt | D-Lys | Phe | Arg | | NH₂ |
| Hmt | D-Orn | Phe | Arg | | NH₂ |
| Hmt | D-Dab | Phe | Arg | | NH₂ |
| Hmt | D-Dap | Phe | Arg | | NH₂ |
| Hmt | D-Arg | Phe | Arg | | NH₂ |

| | | | | | |
|---|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | | |

Peptides which have mu-opioid receptor agonist activity are typically those peptides which have a tyrosine residue or a tyrosine derivative at the N-terminus (*i.e*., the first amino acid position). Suitable derivatives of tyrosine include 2'-methyltyrosine (Mmt); 2',6'-dimethyltyrosine (2'6'-Dmt); 3',5'-dimethyltyrosine (3',5-'Dmt); N,2',6'-trimethyltyrosine (Tmt); and 2'-hydroxy-6'-methyltryosine (Hmt).

Peptides that do not have mu-opioid receptor agonist activity generally do not have a tyrosine residue or a derivative of tyrosine at the N-terminus (*i.e*., amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

The amino acids of the peptides shown in Tables 5 and 6 may be in either the L-or the D-configuration.

In some embodiments, the aromatic-cationic peptides include at least one arginine and/or at least one lysine residue. In some embodiments, the arginine and/or lysine residue serves as an electron acceptor and participates in proton coupled electron transport. Additionally or alternatively, in some embodiments, the aromatic-cationic peptide comprises a sequence resulting in a "charge-ring-charge-ring" configuration such as exists in SS-31. Additionally or alternatively, in some embodiments the aromatic-cationic peptides include thiol-containing residues, such as cysteine and methionine. In some embodiments, peptides including thiol-containing residues directly donate electrons and reduce cytochrome c. In some embodiments, the aromatic-cationic peptides include a vysteine at the N-and/or at the C-terminus of the peptide.

In some embodiments, peptide multimers are provided. For example in some embodiments, dimers are provided, such as an SS-20 dimer: Phe-D-Arg-Phe-Lys-Phe-D-Arg-Phe-Lys. In some embodiments, the dimer is an SS-31 dimer: D-Arg-2',6'-Dmt-Lys-Phe-D-Arg-2',6'-Dmt-Lys-Phe-NH₂. In some embodiments, the multimers are trimers, tetramers and/or pentamers. In some embodiments, the multimers include combinations of different monomer peptides (*e.g*., an SS-20 peptide linked to an SS-31 peptide). In some embodiments, these longer analogs are useful as therapeutic molecules and/or are useful in the sensors, switches and conductors disclosed herein.

In some embodiments, the aromatic-cationic peptides described herein comprise all levorotatory (L) amino acids.

### Peptide Synthesis

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc, New York (1997).

One way of stabilizing peptides against enzymatic degradation is the replacement of an L-amino acid with a D-amino acid at the peptide bond undergoing cleavage. Aromatic cationic peptide analogs are prepared containing one or more D-amino acid residues in addition to the D-Arg residue already present. Another way to prevent enzymatic degradation is *N*-methylation of the α-amino group at one or more amino acid residues of the peptides. This will prevent peptide bond cleavage by *any* peptidase. Examples include: H-D-Arg-2',6'-Dmt-Lys(*N^{α}*Me)-Phe-NH₂; H-D-Arg-2',6'-Dmt-Lys-Phe(*N*Me)-NH₂; H-D-Arg-2',6'-Dmt-Lys(*N^{α}*Me)-Phe(*N*Me)-NH₂; and H-D-Arg(*N^{α}*Me)-2,6-Dmt(*N*Me)-Lys(*N^{α}*Me)*N^{α}*-methylated analogues have lower hydrogen bonding capacity and can be expected to have improved intestinal permeability.

An alternative way to stabilize a peptide amide bond (-CO-NH-) against enzymatic degradation is its replacement with a reduced amide bond (Ψ[CH₂-NH]). This can be achieved with a reductive alkylation reaction between a Boc-amino acid-aldehyde and the amino group of the N-terminal amino acid residue of the growing peptide chain in solid-phase peptide synthesis. The reduced peptide bond is predicted to result in improved cellular permeability because of reduced hydrogen-bonding capacity. Examples include: H-D-Arg-Ψ[CH₂-NH]2',6'-Dmt-Lys-Phe-NH₂,H-D-Arg-2',6'-Dmt-Ψ[CH₂-NH]Lys-Phe-NH₂, H-D-Arg-2',6'-Dmt-LysΨ[CH₂-NH]Phe-NH₂, H-D-Arg-2',6'-Dmt-Ψ[CH₂-NH]Lys-Ψ[CH₂-NH]Phe-NH₂, etc.

### Prophylactic and Therapeutic Uses of Aromatic-Cationic Peptides.

The aromatic-cationic peptides described herein are useful to prevent or treat disease. Specifically, the disclosure provides for both prophylactic and therapeutic methods of treating a subject at risk of (or susceptible to) disease by administering the aromatic-cationic peptides described herein. Accordingly, the present methods provide for the prevention and/or treatment of disease in a subject by administering an effective amount of an aromatic-cationic peptide to a subject in need thereof.

In one aspect, the disclosure provides a method of reducing the number of mitochondria undergoing mitochondrial permeability transition (MPT), or preventing mitochondrial permeability transitioning in a mammal in need thereof, the method comprising administering to the mammal an effective amount of one or more aromatic-cationic peptides described herein. In another aspect, the disclosure provides a method for increasing the ATP synthesis rate in a mammal in need thereof, the method comprising administering to the mammal an effective amount of one or more aromatic-cationic peptides described herein. In yet another aspect, the disclosure provides a method for reducing oxidative damage in a mammal in need thereof, the method comprising administering to the mammal an effective amount of one or more aromatic-cationic peptides described herein.

*Oxidative Damage.* The peptides described above are useful in reducing oxidative damage in a mammal in need thereof. Mammals in need of reducing oxidative damage are those mammals suffering from a disease, condition or treatment associated with oxidative damage. Typically, the oxidative damage is caused by free radicals, such as reactive oxygen species (ROS) and/or reactive nitrogen species (RNS). Examples of ROS and RNS include hydroxyl radical, superoxide anion radical, nitric oxide, hydrogen, hypochlorous acid (HOC1) and peroxynitrite anion. Oxidative damage is considered to be "reduced" if the amount of oxidative damage in a mammal, a removed organ, or a cell is decreased after administration of an effective amount of the aromatic cationic peptides described above. Typically, the oxidative damage is considered to be reduced if the oxidative damage is decreased by at least about 10%, at least about 25%, at least about 50%, at least about 75%, or at least about 90%, compared to a control subject not treated with the peptide.

In some embodiments, a mammal to be treated can be a mammal with a disease or condition associated with oxidative damage. The oxidative damage can occur in any cell, tissue or organ of the mammal. In humans, oxidative stress is involved in many diseases. Examples include atherosclerosis, Parkinson's disease, heart failure, myocardial infarction, Alzheimer's disease, schizophrenia, bipolar disorder, fragile X syndrome and chronic fatigue syndrome.

In one embodiment, a mammal may be undergoing a treatment associated with oxidative damage. For example, the mammal may be undergoing reperfusion. Reperfusion refers to the restoration of blood flow to any organ or tissue in which the flow of blood is decreased or blocked. The restoration of blood flow during reperfusion leads to respiratory burst and formation of free radicals.

In one embodiment, the mammal may have decreased or blocked blood flow due to hypoxia or ischemia. The loss or severe reduction in blood supply during hypoxia or ischemia may, for example, be due to thromboembolic stroke, coronary atherosclerosis, or peripheral vascular disease. Numerous organs and tissues are subject to ischemia or hypoxia. Examples of such organs include brain, heart, kidney, intestine and prostate. The tissue affected is typically muscle, such as cardiac, skeletal, or smooth muscle. For instance, cardiac muscle ischemia or hypoxia is commonly caused by atherosclerotic or thrombotic blockages which lead to the reduction or loss of oxygen delivery to the cardiac tissues by the cardiac arterial and capillary blood supply. Such cardiac ischemia or hypoxia may cause pain and necrosis of the affected cardiac muscle, and ultimately may lead to cardiac failure.

The methods can also be used in reducing oxidative damage associated with any neurodegenerative disease or condition. The neurodegenerative disease can affect any cell, tissue or organ of the central and peripheral nervous system. Examples of such cells, tissues and organs include, the brain, spinal cord, neurons, ganglia, Schwann cells, astrocytes, oligodendrocytes and microglia. The neurodegenerative condition can be an acute condition, such as a stroke or a traumatic brain or spinal cord injury. In another embodiment, the neurodegenerative disease or condition can be a chronic neurodegenerative condition. In a chronic neurodegenerative condition, the free radicals can, for example, cause damage to a protein. An example of such a protein is amyloid β-protein. Examples of chronic neurodegenerative diseases associated with damage by free radicals include Parkinson's disease, Alzheimer's disease, Huntington's disease and Amyotrophic Lateral Sclerosis (also known as Lou Gherig's disease).

Other conditions which can be treated include preeclampsia, diabetes, and symptoms of and conditions associated with aging, such as macular degeneration, wrinkles.

*Mitochondrial Permeability Transitioning.* The peptides described above are useful in treating any disease or condition that is associated with mitochondria permeability transitioning (MPT). Such diseases and conditions include, but are not limited to, ischemia and/or reperfusion of a tissue or organ, hypoxia and any of a number of neurodegenerative diseases. Mammals in need of inhibiting or preventing of MPT are those mammals suffering from these diseases or conditions.

*Determination of the Biological Effect of the Aromatic-Cationic Peptide-Based Therapeutic.* In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect in preventing or treating disease. Compounds for use in therapy can be tested in suitable animal model systems including, but not limited to rats, mice, chicken, pigs, cows, monkeys, rabbits, and the like, prior to testing in human subjects. Similarly, for *in vivo* testing, any of the animal model systems known in the art can be used prior to administration to human subjects.

*Prophylactic Methods.* In one aspect, the invention provides a method for preventing, in a subject, disease by administering to the subject an aromatic-cationic peptide that prevents the initiation or progression of the condition. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the aberrancy, such that a disease or disorder is prevented or, alternatively, delayed in its progression. The appropriate compound can be determined based on screening assays described above.

*Therapeutic Methods.* Another aspect of the technology includes methods of treating disease in a subject for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease.

### Modes of Administration and Effective Dosages

Any method known to those in the art for contacting a cell, organ or tissue with a peptide may be employed. Suitable methods include *in vitro, ex vivo, or in vivo* methods. *In vivo* methods typically include the administration of an aromatic-cationic peptide, such as those described above, to a mammal, suitably a human. When used *in vivo* for therapy, the aromatic-cationic peptides are administered to the subject in effective amounts (*i.e*., amounts that have desired therapeutic effect). The dose and dosage regimen will depend upon the degree of the injury in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (*e.g*., salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (*e.g.,* citric, gluconic, glycolic, lactic, lactobionic, malic, and tartaric acids), aliphatic monocarboxylic acids (*e.g*., acetic, butyric, formic, propionic and trifluoroacetic acids), amino acids (*e.g*., aspartic and glutamic acids), aromatic carboxylic acids (*e.g*., benzoic, p-chlorobenzoic, diphenylacetic, gentisic, hippuric, and triphenylacetic acids), aromatic hydroxyl acids (*e.g*., o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g*., fumaric, maleic, oxalic and succinic acids), glucoronic, mandelic, mucic, nicotinic, orotic, pamoic, pantothenic, sulfonic acids (*e.g*., benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like. In some embodiments, the salt is an acetate salt. Additionally or alternatively, in other embodiments, the salt is a trifluoroacetate salt.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (*e.g*., intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g*., vials of drug, vials of diluent, syringes and needles) for a treatment course (*e.g*., 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed my iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. (*See* Lichtenberg et al., Methods Biochem. Anal., 33:337-462 (1988); Anselem et al., Liposome Technology, CRC Press (1993)). Liposomal formulations can delay clearance and increase cellular uptake (*See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34(7-8):915-923 (2000)). A polymer formulation for human growth hormone (hGH) has been used in clinical trials. *(See* Kozarich and Rich, Chemical Biology, 2:548-552 (1998)).

Examples of polymer microsphere sustained release formulations are described in PCT publication WO 99/15154 (Tracy et al.)*,* U.S. Pat. Nos. 5,674,534 and 5,716,644 (both to Zale et al.)*,* PCT publication WO 96/40073 (Zale et al.)*,* and PCT publication WO 00/38651 (Shah et al.)*.* U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, *e.g*., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹² to 10⁻⁶ molar, *e.g*., approximately 10⁻⁷ molar. This concentration may be delivered by systemic doses of 0.01 to 100 mg/kg or equivalent dose by body surface area. The schedule of doses would be optimized to maintain the therapeutic concentration at the target tissue, most preferably by single daily or weekly administration, but also including continuous administration (*e.g*., parenteral infusion or transdermal application).

In some embodiments, the dosage of the aromatic-cationic peptide is provided at about 0.001 to about 0.5 mg/kg/h, suitably from about 0.01 to about 0.1 mg/kg/h. In one embodiment, the is provided from about 0.1 to about 1.0 mg/kg/h, suitably from about 0.1 to about 0.5 mg/kg/h. In one embodiment, the dose is provided from about 0.5 to about 10 mg/kg/h, suitably from about 0.5 to about 2 mg/kg/h.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In a preferred embodiment, the mammal is a human.

### Aromatic-cationic peptides in electron transfer

Mitochondrial ATP synthesis is driven by electron flow through the electron transport chain (ETC) of the inner mitochondrial membrane (IMM). Electron flow through the chain can be described as a series of oxidation/reduction processes. Electrons pass from electron donors (NADH or QH2), through a series of electron acceptors (Complexes I-IV), and ultimately to the terminal electron acceptor, molecular oxygen. Cytochrome c (cytochrome c), which is loosely associated with the IMM, transfers electrons between Complexes III and IV.

Rapid shunting of electrons through the ETC is important for preventing short-circuiting that would lead to electron escape and generation of free radical intermediates. The rate of electron transfer (ET) between an electron donor and electron acceptor decreases exponentially with the distance between them, and superexchange ET is limited to 20Å. Long-range ET can be achieved in a multi-step electron hopping process, where the overall distance between donor and acceptor is split into a series of shorter, and therefore faster, ET steps. In the ETC, efficient ET over long distances is assisted by cofactors that are strategically localized along the IMM, including FMN, FeS clusters, and hemes. Aromatic amino acids such as Phe, Tyr and Trp can also facilitate electron transfer to heme through overlapping *π* clouds, and this was specifically shown (*see* experimental examples) for cytochrome c. Amino acids with suitable oxidation potential (Tyr, Trp, Cys, Met) can act as stepping stones by serving as intermediate electron carriers. In addition, the hydroxyl group of Tyr can lose a proton when it conveys an electron, and the presence of a basic group nearby, such as Lys, can result in proton-coupled ET which is even more efficient.

Overexpression of catalase targeted to mitochondria (mCAT) has been shown to improve aging (*e.g*., reduce the symptoms) and prolong lifespan in mice. These examples identify "druggable" chemical compounds that can reduce mitochondrial oxidative stress and protect mitochondrial function. As mitochondria are the major source of intracellular reactive oxygen species (ROS), the antioxidant must be delivered to mitochondria in order to limit oxidative damage to mitochondrial DNA, proteins of the electron transport chain (ETC), and the mitochondrial lipid membranes. We discovered a family of synthetic aromatic-cationic tetrapeptides that selectively target and concentrate in the inner mitochondrial membrane (IMM). Some of these peptides contain redox-active amino acids that can undergo one-electron oxidation and behave as mitochondria-targeted antioxidants. The peptides disclosed herein, such as the peptide D-Arg-2'6'-Dmt-Tyr-Lys-Phe-NH₂ reduces mitochondrial ROS and protect mitochondrial function in cellular and animal studies. Recent studies show that this peptide can confer protection against mitochondrial oxidative stress comparable to that observed with mitochondrial catalase overexpression. Although radical scavenging is the most commonly used approach to reduce oxidative stress, there are other potential mechanisms that can be used, including facilitation of electron transfer to reduce electron leak and improved mitochondrial reduction potential.

Abundant circumstantial evidence indicates that oxidative stress contributes to many consequences of normal aging and several major diseases, including cardiovascular diseases, diabetes, neurodegenerative diseases, and cancer. Oxidative stress is generally defined as an imbalance of prooxidants and antioxidants. However, despite a wealth of scientific evidence to support increased oxidative tissue damage, large-scale clinical studies with antioxidants have not demonstrated significant health benefits in these diseases. One of the reasons may be due to the inability of the available antioxidants to reach the site of prooxidant production.

The mitochondrial electron transport chain (ETC) is the primary intracellular producer of ROS, and mitochondria themselves are most vulnerable to oxidative stress. Protecting mitochondrial function would therefore be a prerequisite to preventing cell death caused by mitochondrial oxidative stress. The benefits of overexpressing catalase targeted to mitochondria (mCAT), but not peroxisomes (pCAT), provided proof-of-concept that mitochondria-targeted antioxidants would be necessary to overcome the detrimental effects of aging. However, adequate delivery of chemical antioxidants to the IMM remains a challenge.

One peptide analog, D-Arg-2'6'-Dmt-Tyr-Lys-Phe-NH₂, possesses intrinsic antioxidant ability because the modified tyrosine residue is redox-active and can undergo one-electron oxidation. We have shown that this peptide can neutralize H₂O₂, hydroxyl radical, and peroxynitrite, and inhibit lipid peroxidation. The peptide has demonstrated remarkable efficacy in animal models of ischemia-reperfusion injury, neurodegenerative diseases, and metabolic syndrome.

The design of the mitochondria-targeted peptides incorporates and enhances one or more of the following modes of action: (i) scavenging excess ROS, (ii) reducing ROS production by facilitating electron transfer, or (iii) increasing mitochondrial reductive capacity. The advantage of peptide molecules is that it is possible to incorporate natural or unnatural amino acids that can serve as redox centers, facilitate electron transfer, or increase sulfydryl groups while retaining the aromatic-cationic motif required for mitochondria targeting.

### Aromatic-cationic peptides for electronic and optical sensing

As illustrated by the examples, changing the concentration of aromatic-cationic peptides disclosed herein, including peptides that comprise the amino acid sequence Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), in a sample alters the electrical and photoluminescent properties of cytochrome c. Specifically, increasing the aromatic-cationic peptide concentration relative to cytochrome c causes the conductivity and photoluminescent efficiency of cytochrome c to increase. Suitable ranges of aromatic-cationic peptide concentration include, but are not limited to, 0-500 mM; 0-100 mM; 0-500 um; 0-250 µm; and 0-100 µm.

These changes in conductivity and photoluminescent efficiency can be exploited for conducting, sensing, switching, and/or enhancing the emission of light from cytochrome c as described below. For example, cytochrome c, aromatic-cationic peptides, and/or peptide-doped cytochrome c can be used to make and/or enhance sensors; pressure/temperature/pH-to-current transducers; field-effect transistors, including light-emitting transistors; light-emitting devices, such as diodes and displays; batteries; and solar cells. The aromatic-cationic peptide concentration level (e.g, in cytochrome c) can also be spatially varied to create regions with different band gaps; these variations in band gap can be used to make heterojunctions, quantum wells, graded band gap regions, etc., that can be incorporated into the aforementioned sensors, transistors, diodes, and solar cells to enhance their performance.

### Cytochrome c Sensors Doped with Aromatic-Cationic Peptides

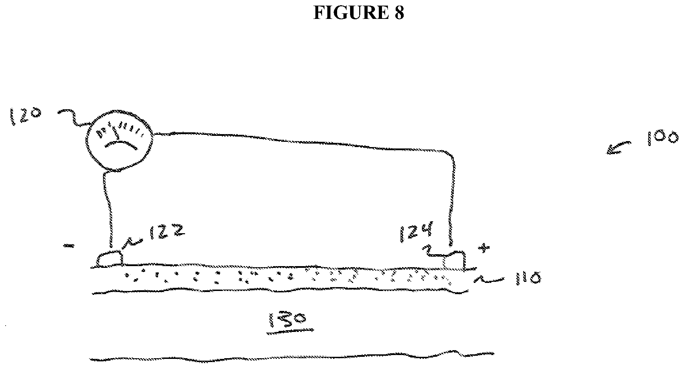

FIG. 8 shows an example sensor 100 that detects changes in pH and/or temperature of a test substrate 130 by measuring the change in conductivity (resistance) of a layer 110 of cytochrome c doped with any of the peptides disclosed herein, for example Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31). As the temperature and/or pH of the substrate 130 changes, the aromatic-cationic peptide diffuses into or out of the peptide-doped cytochrome c layer 110, which in turn causes the conductivity of the peptide-doped cytochrome c layer 110 to change. A meter 120 measures the variation in conductivity by applying an electrical potential (voltage) to the cytochrome c layer 110 via an anode 122 and a cathode 124. When the conductivity goes up, the current flowing between the anode 122 and the cathode 124 increases. When the conductivity goes down, the current flowing between the anode 122 and the cathode 124 decreases. Alternative sensors may include additional electrical terminals (i.e., anodes and cathodes) for more sensitive resistance measurements. For example, alternative sensors may include four electrical terminals for Kelvin sensing measurements of resistance.

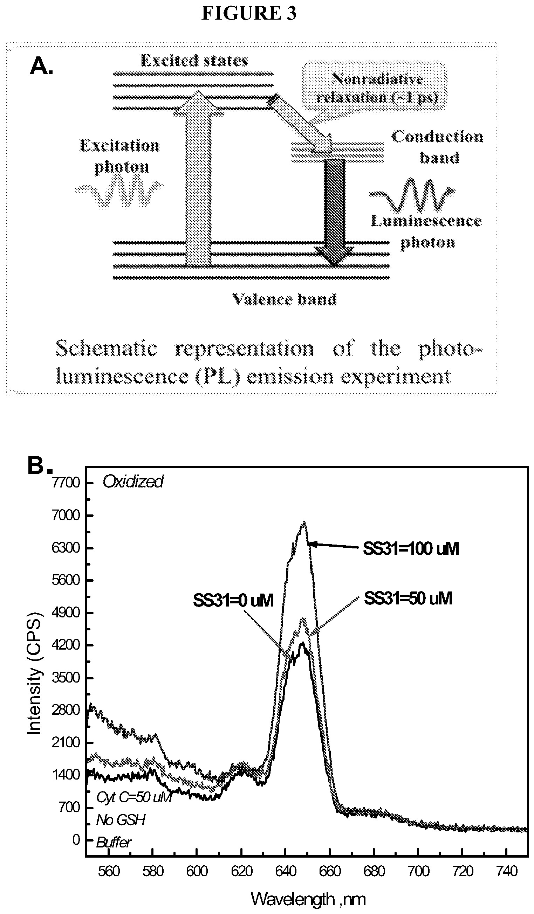

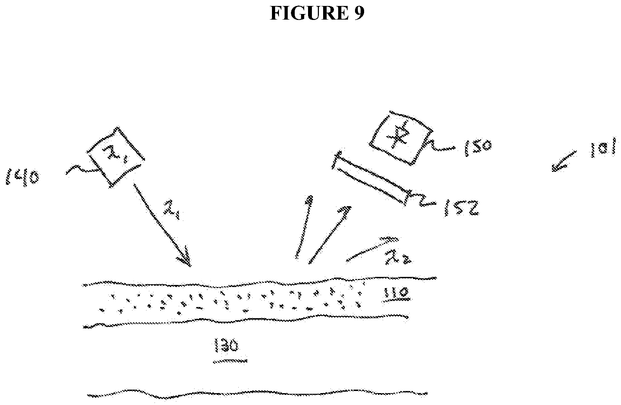

FIG. 9 shows an alternative sensor 101 that detects changes in pH and/or temperature of the test substrate 130 by measuring the change in photoluminescence of the peptide-doped cytochrome c layer 110. A light source 140, such as a laser or light-emitting diode (LED), illuminates the peptide-doped cytochrome c layer 110 at an excitation wavelength, such as 532.8 nm. As shown in FIG. 3A, illumination of the peptide-doped cytochrome c layer 110 at the excitation wavelength excites an electron from a valence band to an excited state. (As understood by those skilled in the art, the gap between the valence band and the excited state is proportional to the excitation wavelength.) After a short relaxation time, the electron decays from the excited state to a conduction band. When the electron relaxes to valence band from the conduction band, the peptide-doped cytochrome c layer 110 emits a photon at a luminescence wavelength, such as 650 nm, fixed by the gap between the valence and conduction bands.

As shown in FIG. 3B, the intensity of light emitted by cytochrome c for a constant excitation intensity (from the source 140) varies nonlinearly with the aromatic-cationic peptide concentration: increasing the aromatic-cationic peptide concentration from 0 µM to 50 µM increases the emitted intensity at the luminescence wavelength from about 4200 CPS to about 4900 CPS, whereas doubling the aromatic-cationic peptide concentration from 50 µM to 100 µM increases the emitted intensity at the luminescence wavelength from about 4900 CPS to about 7000 CPS. Thus, as the aromatic-cationic peptide concentration in the peptide-doped cytochrome c layer 110 varies due to changes in the pH and/or temperature of the test substrate 130, the intensity at the luminescence wavelength varies as well. Detecting this change in intensity with a photodetector 150 yields an indication of the pH and/or temperature of the test substrate 130.

In some cases, changes in aromatic-cationic peptide concentration may cause changes in the wavelength of the luminescent emission instead of or in addition to changes in the intensity of the luminescent emission. These changes in emission wavelength can be detected by filtering emitted light with a filter 152 disposed between the peptide-doped layer 110 and the detector 150. The filter 152 transmits light within a passband and reflects and/or absorbs light outside the passband. If the emission wavelength falls outside the passband due to pH-and/or temperature-induced changes in peptide concentration, then the detector 150 does not detect any light, an effect that can be exploited to determine changes in peptide concentration. Alternatively, aromatic-cationic peptide-induced changes in luminescence wavelength can be measured by analyzing the spectrum of the unfiltered emission, e.g., with an optical spectrum analyzer (not shown) instead of a photodetector 150.

Those skilled in the art will readily appreciate that the aromatic-cationic peptides disclosed herein, such as peptide Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), can also be used to enhance and/or tune the wavelength of light emitted from optically and/or electrically stimulated cytochrome c. For example, doping cytochrome c at a peptide concentration of 100 µM nearly doubles the intensity of light emitted at 650 nm as shown by FIG. 3B. Thus, the sensor 101 of FIG. 9 can also be used as an enhanced light-emitting element. Unlike semiconductor LEDs and displays, an enhanced light-emitting element based on peptide-doped cytochrome c could be made in arbitrary shapes and on flexible substrates. In addition, the peptide concentration can be set to provide a desired level and/or wavelength of illumination.

Sensors made using cytochrome c, aromatic-cationic peptides, and/or peptide-doped cytochrome c can be used to detect changes in pressure, temperature, pH, applied field, and/or other properties that affect conductivity. For example, sensors 100 and 101 can be used to detect changes in pressure that affect the concentration of aromatic-cationic peptide in the cytochrome c; as pressure changes cause aromatic-cationic peptide to diffuse into the cytochrome c, the conductivity and/or emission intensity increases, and vice versa. Changes in temperature and pH that affect the peptide concentration in the cytochrome c produce similar results. Applied fields, such as electromagnetic fields, that change the peptide concentration in the cytochrome c also cause the measured conductivity, emission intensity, and emission wavelength to change.

Cytochrome c sensors doped with aromatic-cationic peptides can also be used to sense biological and/or chemical activity as disclosed herein. For example, exemplary sensors may be used to identify other molecules and/or atoms that are coupled to the aromatic-cationic peptide and/or the cytochrome c and that change the electrical and luminescent properties of the peptide-doped cytochrome c. In some cases, a single molecule of cytochrome c doped with a single peptide molecule, such as a molecule of Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), may be able to detect minute variations in pressure, temperature, pH, applied field, etc. caused by the aromatic-cationic peptide molecule binding itself to or releasing itself from the cytochrome c molecule. Single-molecule sensors (and/or multiple-molecule sensors) may be arranged in regular (e.g., periodic) or irregular arrays for detecting any of the aforementioned qualities in applications including, but not limited to, enzymatic analysis (e.g., glucose and lactate assays), DNA analysis (e.g., polymerase chain reaction and high-throughput sequencing), and proteomics.

### Cytochrome c Doped with Aromatic-Cationic Peptides In Microfluidics

In addition, peptide-doped cytochrome c sensors can be used in microfluidic and optofluidic devices, e.g., to transduce variations in pressure, temperature, pH, applied field, etc. into electrical currents and/or voltages for use in hybrid biological/chemical/electronic processors. They can also be used in microfluidic and optofluidic devices, such as those described in U.S. Patent Application Publication No. 2009/0201497, U.S. Patent Application Publication No. 2010/0060875, and U.S. Patent Application Publication No. 2011/0039730, each of which is incorporated by reference herein in its entirety.

Optofluidics refers to manipulation of light using fluids, or vice-versa, on the micro to nano meter scale. By taking advantage of the microfluidic manipulation, the optical properties of the fluids can be precisely and flexibly controlled to realize reconfigurable optical components which are otherwise difficult or impossible to implement with solid-state technology. In addition, the unique behavior of fluids on micro/nano scale has given rise to the possibility to manipulate the fluid using light. Applications of optofluidic devices based on cytochrome c doped with aromatic-cationic peptide(s) include, but are not limited to: adaptive optical elements; detection using microresonators; fluidic waveguides; fluorescent microfluidic light sources; integrating nanophotonics and microfluidics; micro-spectroscopy; microfluidic quantum dot bar-codes; microfludics for nonlinear optics applications; optofluidic microscopy; optofluidic quantum cascade lasers for reconfigurable photonics and on-chip molecular detectors; optical memories using nanoparticle cocktails; and test tube microcavity lasers for integrated opto-fluidic applications.

Sensors comprising cytochrome c doped with aromatic-cationic peptide(s) can be used in microfluidic processors to transduce pressure variations due to changes in fluid flow into variations in electrical and/or optical signals that can be readily detected using conventional electrical detectors and photodetectors as described above. Peptide-doped cytochrome c transducers can be used to control microfluidic pumps, processors, and other devices, including tunable microlens arrays.

### Cytochrome c doped with Aromatic-Cationic Peptide(s) for Switches And Transistors

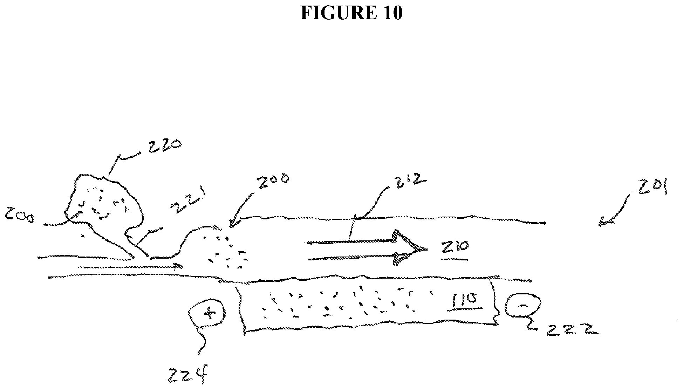

Cytochrome c doped with aromatic-cationic peptide(s) can also be used as, or in an electrical or optical switch, *e.g*., switch 201 shown in FIG. 10. The switch 201 includes a reservoir 220, which holds an aromatic-cationic peptide 200, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), in fluid communication with cytochrome c or peptide-doped cytochrome c 110 via a conduit 221 and a channel 210. In operation, the conduit 221 is opened to allow the peptide 200 to flow in direction 212 into the channel 210. The switch 201 is actuated by creating a temperature and/or pH gradient across the boundary between the channel 210 and the cytochrome c 130. Depending on the direction of the gradient, peptide 200 diffuses into or out of the cytochrome c 130, which causes the conductivity and photoluminescent qualities to change as described above. Changes in conductivity due to fluctuations in peptide concentration can be used to regulate current flow between an anode 222 and a cathode 224.

The switch 201 shown in FIG. 10 act as an organic field-effect transistor (OFET): it regulates current flow in response to changes in a "field" corresponding to the temperature and/or pH gradient across the boundary between the channel 210 and the cytochrome c 130. Each transistor includes a cytochrome c channel layer or a cytochrome c channel layer doped with aromatic-cationic peptide, a gate, a source and a drain. The channel layer is disposed above a lower substrate. The source and the drain are disposed above the channel layer and respectively contact with the two opposite sides of the channel layer. The gate is disposed above the channel layer and positioned between the source and the drain. The above organic electroluminescent device is electrically connected to the drain for receiving the current outputted from the source via the channel layer and emitting according to the magnitude of the current.

Compared to conventional transistors, transistors of the present invention, such as peptide-doped cytochrome c OFETs may be simple to manufacture. Conventional inorganic transistors require high temperatures (e.g., 500-1,000°C), but OFETs can be made between room temperature and 200°C. OFETs can even be formed even on a plastic substrate, which is vulnerable to heat. OFETs can be used to realize light, thin, and flexible device elements, allowing them to be used in a variety of unique devices, such as flexible displays and sensors.

OFETs can be used to implement the fundamental logic operations necessary for digital signal processing. For example, transistors can be used to create (nonlinear) logic gates, such as NOT and NOR gates, that can be coupled together for processing digital signals. Peptide-doped cytochrome c transistors can be used in applications including but not limited to emitter followers (e.g., for voltage regulation), current sources, counters, analog-to-digital conversion, etc., and in both general-purpose computing and application-specific processing, such as processing for computer networking, wireless communication (e.g., software-defined radio), etc. See P. Horowitz and W. Hill's "The Art of Electronics," which is incorporated herein by reference in its entirety, for more applications of transistors.

Transistors can also be used to amplify signals by translating a small change in one property, e.g., pH, into a large change in another property, e.g., conductivity; as well understood, amplification can be used for a variety of applications, including wireless (radio) transmission, sound reproduction, and (analog) signal processing. Peptide-doped cytochrome c transistors can also be used to make operational amplifiers (op amps), which are used in inverting amplifiers, non-inverting amplifiers, feedback loops, oscillators, etc. For more on organic transistors, see U.S. Patent No. 7,795,611; U.S. Patent No. 7,768,001; U.S. Patent No. 7,126,153; and U.S. Patent No. 7,816,674, each of which is incorporated herein by reference in its entirety.

### Cytochrome c Doped with Aromatic-Cationic Peptides for Random Access Memory

Transistors based on cytochrome c and/or cytochrome c doped with an aromatic-cationic peptides as disclsed herein, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), can also be used to implement memory, such as static or dynamic random access memory (RAM), that stores information for use in digital computing. As well understood, six transistors can coupled together to form a static RAM (SRAM) cell that stores one bit of information without the need for periodic refreshing. Transistors based on cytochrome c and/or cytochrome c doped with aromatic-cationic peptides can also be used to implement other types of memory, including dynamic random access memory (DRAM), for digital computation. As well understood, RAM can be used to implement digital computing for applications such as those described above.

Peptide-doped cytochrome c transistors may be formed in programmable or preprogrammed biological arrays much like conventional transistors are formed in integrated circuits. If the change in conductivity (resistivity) of cytochrome c due to peptide activity is high enough, an example transistor (switch) can be made of a single cytochrome c molecule doped with a single peptide molecule. Arrays of single-molecule cytochrome c transistors can be formed to create incredibly small, densely packed logic circuits.

### Cytochrome c Doped with Inventive Aromatic-Cationic Peptides for Light-Emitting Transistors

Cytochrome c and/or cytochrome c doped with with an aromatic-cationic peptide as disclosed herein, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31) can also be used to make organic light-emitting transistors (OLETs) that could lead to cheaper digital displays and fast-switching light sources on computer chips. An OLET-based light source switches much faster than a diode, and because of its planar design it could be more easily integrated onto computer chips, providing faster data transmission across chips than copper wire. The key to higher efficiency is a three-layer structure, with thin films stacked on top of one another. Current flows horizontally through the top and bottom layers-one carrying electrons and the other holes-while carriers that wander into the central layer recombine and emit photons. As the location of the joint region in the channel is dependent on the gate and drain voltages, the emission region can be tuned.

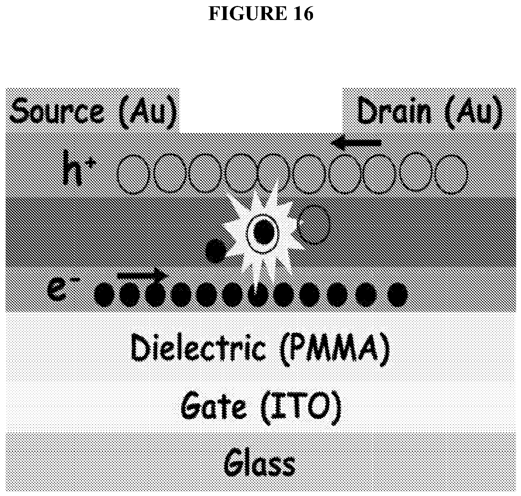

An example OLET, such as the OLET shown in FIG. 16, may be constructed on a transparent (e.g., glass) substrates coated with a indium tin oxide layer, which serves as the transistor's gate, coated with a layer of poly(methyl methacrylate) (PMMA), a common dielectric material. A multi-layer organic structure, which may include a film of an electron-transporting material (e.g., peptide-doped cytochrome c), a film of emissive material, and a hole-transporting material is deposited onto the PMMA. Finally, metal contacts are deposited on top of the organic structure to provide a source and a drain. The light in the OLET is emitted as a stripe along the emissive layer, rather than up through the contacts as in an OLED. The shape of the emissive layer can be varied to make it easier to couple the emitted light into optical fibers, waveguides, and other structures.

The organic light-emitting transistor (OLET) developed by Hepp et al. in 2003 operates in unipolar p-type mode and produces green electroluminescence close to the gold drain electrode (electron injection). The emission region of the Hepp device, however, could not be modulated due to the unipolar operation mode. Balanced ambipolar transport is highly desirable for improving the quantum efficiency of OLETs, and is important to both single-component and heterostructure transistors.

Ambipolar OLETs may be based on a heterostructure of hole-transport material and electron-transport material, such as peptide-doped cytochrome c. The light intensity of an ambipolar OLET can be controlled by both the drain-source voltage and the gate voltage. The carrier mobility and electroluminescent properties of OLETs based on the same materials (e.g., peptide-doped cytochrome c) can be tuned by changing the ratio of the two components . Higher concentration of hole-transport material may result in non-light-emitting ambipolar FETs, whereas a higher concentrations of peptide-doped cytochrome c (or of peptide concentrations in cytochrome c) can result in light-emitting unipolar n-channel FETs.

OLETs based on two-component layered structures can be realized by sequentially depositing hole-transport material and electron-transport material. Morphological analysis indicates a continuous interface between the two organic films, which is crucial for controlling the quality of the interface and the resulting optoelectronic properties of the OLETs. An overlapping p-n heterostructure can be confined inside the transistor channel by changing the tilt angle of the substrate during the sequential deposition process. The emission region (i.e., the overlapping region) is kept away from the hole and electron source electrodes, avoiding exciton and photon quenching at the metal electrodes. OLETs can also be realized in alternative heterostructures, including a vertical combination static induction transistor with an OLED, top-gate-type OLETs similar to a top-gate static induction transistor or triode, and OLETs having a laterally arranged heterojunction structure and diode/FET hybrid. Further details of organic light-emitting transistors can be found in U.S. Patent No. 7,791,068 to Meng et al.*,* and U.S. Patent No. 7,633,084 to Kido et al.*,* each of which is incorporated herein by reference in its entirety.

Alternatively, or in addition, the aromatic-cationic peptide concentration can be used to regulate the intensity and/or wavelength of light emitted by the cytochrome c 110. Suitable ranges of aromatic-cationic peptide concentration include, but are not limited to, 0-500 mM; 0-100 mM; 0-500 µm; 0-250 µm; and 0-100 µm. In fact, the nonlinear change in emitted intensity shown in FIG. 3B indicates that peptide-doped cytochrome c 110 is well-suited for binary (digital) switching: when the peptide concentration is below a predetermined threshold, *e.g.,* 50 µM, the emitted intensity is below a given level, *e.g.,* 5000 CPS. At aromatic-cationic peptide concentrations above the threshold, *e.g*., 100 µM, the emitted intensity jumps, *e.g*., to about 7000 CPS. This nonlinear behavior can be exploited to detect or respond to a corresponding change in pH or temperature of the cytochrome c 110 and/or any layers or substances in thermal and/or fluid communication with the cytochrome c 110.

### Cytochrome c Doped with Aromatic-Cationic Peptides for Light-Emitting Diodes And Electroluminescent Displays

Cytochrome c and/or cytochrome c doped with an aromatic-cationic peptide as disclosed herein, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31) can be used in organic light-emitting diodes (OLEDs) and electroluminescent displays. OLEDs are useful in a variety of consumer products, such as watches, telephones, lap-top computers, pagers, cellular phones, digital video cameras, DVD players, and calculators. Displays containing OLEDs have numerous advantages over conventional liquid-crystal displays (LCDs). Because OLED-based display do not require backlights, they can display deep black levels and achieve relatively high contrast ratios, even at wide viewing angles. They can also be thinner, more efficient, and brighter than LCDs, which require heavy, power-hungry backlights. As a result of these combined features, OLED displays are lighter in weight and take up less space than LCD displays.

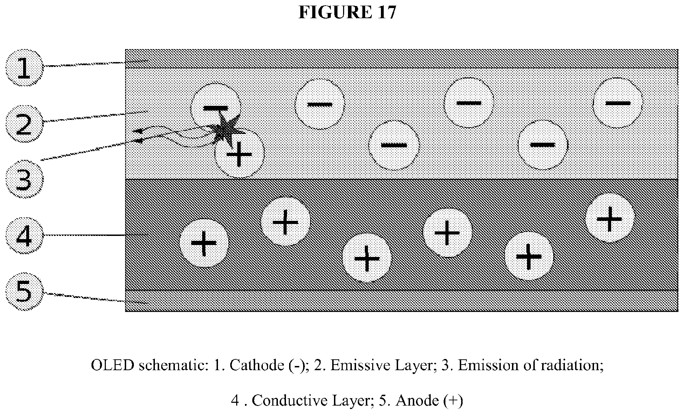

OLEDs typically comprise a light-emitting element interposed between two electrodes - an anode and a cathode - as shown in FIG. 17. The light-emitting element typically comprises a stack of thin organic layers comprising a hole-transport layer, an emissive layer, and an electron-transport layer. OLEDs can also contain additional layers, such as a hole-injection layer and an electron-injection layer. Doping a cytochrome c emissive layer with an aromatic-cationic peptide (and possibly other dopants as well) can enhance the electroluminscent efficiency of the OLED and control color output. Peptide-doped cytochrome c can also be used as the electron-transport layer.

In OLEDs, a layer of cytochrome c doped with aromatic-cationic peptide, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), is coated (e.g., spin-coated) or otherwise disposed between two electrodes, at least one of which is transparent. For example, OLED-based displays may screen-printed, printed with ink-jet printers, or deposited using roll-vapour deposition onto any suitable substrate, including both rigid and flexible substrates. Typical substrates are at least partially transmissive in the visible region of the electromagnetic spectrum. For example, transparent substrate (and electrode layers) may have a percent transmittance of at least 30%, alternatively at least 60%, alternatively at least 80%, for light in the visible region (400 nm to 700 nm) of the electromagnetic spectrum. Examples of substrates include, but are not limited to, semiconductor materials such as silicon, silicon having a surface layer of silicon dioxide, and gallium arsenide; quartz; fused quartz; aluminum oxide; ceramics; glass; metal foils; polyolefins such as polyethylene, polypropylene, polystyrene, and polyethyleneterephthalate; fluorocarbon polymers such as polytetrafluoroethylene and polyvinylfluoride; polyamides such as Nylon; polyimides; polyesters such as poly(methyl methacrylate) and poly(ethylene 2,6-naphthalenedicarboxylate); epoxy resins; polyethers; polycarbonates; polysulfones; and polyether sulfones.

Typically, at least one surface of the substrate is coated with a first electrode, which may be a transparent material, such as indium tin oxide (ITO) or any other suitable material. The first electrode layer can function as an anode or cathode in the OLED. The anode is typically selected from a high work-function (>4 eV) metal, alloy, or metal oxide such as indium oxide, tin oxide, zinc oxide, indium tin oxide (ITO), indium zinc oxide, aluminum-doped zinc oxide, nickel, and gold. The cathode can be a low work-function (<4 eV) metal such as Ca, Mg, and Al; a high work-function (>4 eV) metal, alloy, or metal oxide, as described above; or an alloy of a low-work function metal and at least one other metal having a high or low work-function, such as Mg-Al, Ag-Mg, Al-Li, In-Mg, and Al-Ca. Methods of depositing anode and cathode layers in the fabrication of OLEDs, such as evaporation, co-evaporation, DC magnetron sputtering ,or RF sputtering, are well known in the art.

The active layers, including the cytochrome c and/or cytochrome c layers doped with aromatic-cationic peptides, are coated onto the transparent electrode to form a light-emitting element. The light-emitting element comprises a hole-transport layer and an emissiveve/electron-transport layer, wherein the hole-transport layer and the emissive/electron-transport layer lie directly on one another, and the hole-transport layer comprises a cured polysiloxane, described below. The orientation of the light-emitting element depends on the relative positions of the anode and cathode in the OLED. The hole-transport layer is located between the anode and the emissive/electron-transport layer and the emissive/electron-transport layer is located between the hole-transport layer and the cathode. The thickness of the hole-transport layer can be from 2 to 100 nm, alternatively from 30 to 50 nm. The thickness of the emissive/electron-transport layer can be from 20 to 100 nm, alternatively from 30 to 70 nm.

OLED displays can be driven with either passive-matrix or active-matrix addressing schemes, both of which are well known. For example, an OLED display panel may include an active matrix pixel array and several thin film transistors (TFTs), each of which may be implemented as a peptide-doped cytochrome c transistor (as described above). The active matrix pixel array is disposed between the substrates that contain the active layers. The active matrix pixel array includes several pixels. Each pixel is defined by a first scan line and its adjacent second scan line as well as a first data line and its adjacent second data line both of which are disposed on the lower substrate. TFTs disposed inside the non-display regions of the pixels are electrically connected to the corresponding scan and data lines. Switching the TFTs in the pixels with the scan and data lines causes the corresponding pixels to turn on (*i.e*., to emit light).

In addition, the active layer (*e.g*., the cytochrome c and/or peptide-doped cytochrome c) can be arranged in nearly arbitrary shapes and sizes, and can be patterned into arbitrary shapes. They may also be further doped to generate light at specific wavelengths. Further details of organic light-emitting diodes and organic light-emitting displays can be found in U.S Patent No. 7,358,663; U.S Patent No. 7,843,125; U.S Patent No. 7,550,917; U.S Patent No. 7,714,817; and U.S Patent No. 7,535,172, each of which is incorporated herein by reference in its entirety.

### Cytochrome c Doped with Aromatic-Cationic Peptides for Heterojunctions

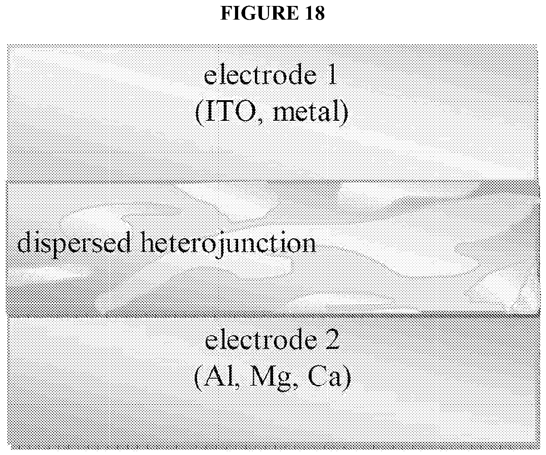

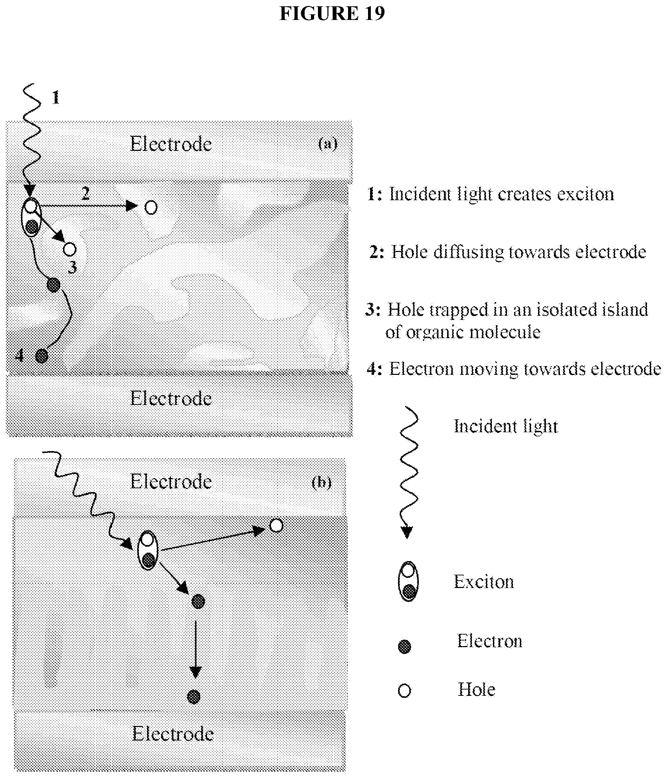

The concentration level of aromatic-cationic peptide in the cytochrome c active layer(s) may also be varied as a function of space and/or time to provide a heterojunction, which is an interface between two semiconductor materials of differing energy gap, as described in U.S. Patent No. 7,897,429, which is incorporated herein by reference in its entirety, and illustrated in the photovoltaic cells of FIGS. 18 and 19. Suitable ranges of aromatic-cationic peptide concentration include, but are not limited to, 0-500 mM; 0-100 mM; 0-500 µM; 0-250 µM; and 0-100 µM. For example, heterojunctions can be used to create multiple quantum well structure for enhanced emission in OLEDs and other devices. Organic heterojunctions have been drawing increasing attention following the discovery of high conductivity in organic heterojunction transistors constructed with active layers of p-type and n-type thin crystalline films. In contrast with the depletion layers that form in inorganic heterojunctions, electron-and hole-accumulation layers can be observed on both sides of organic heterojunction interfaces. Heterojunction films with high conductivity can be used as charge injection buffer layers and as a connecting unit for tandem diodes. Ambipolar transistors and light-emitting transistors (described above) can be realized using organic heterojunction films as active layers.

Organic heterostructures can be used in OLEDs (discussed above), OFETs (discussed above), and organic photovoltaic (OPV) cells (discussed below) to improve device performance. In a typical double-layer OLED structure, the organic heterojunction reduces the onset voltage and improves the illumination efficiency. Organic heterojunctions can also be used to improve the power conversion efficiency of OPV cells by an order of magnitude over single-layer cells Ambipolar OFETs (discussed above), which require that both electrons and holes be accumulated and transported in the device channel depending on the applied voltage, can be realized by introducing organic heterostructures, including peptide-doped cytochrome c, as active layers. Organic heterostructures have an important role in the continued development of organic electronic devices.

Organic heterostructures can also be used as buffer layers in OFETs to improve the contact between the electrodes and the organic layers. For example, a thin layer of cytochrome c and/or peptide-doped cytochrome c can be inserted between the electrodes and the semiconducting layer, resulting in better carrier injection and improved mobility. Organic heterojunctions with high conductivity (*e.g*., due to the use of cytochrome c doped with aromatic-cationic peptide) can also be used as a buffer layer in OFETs to improve the contact between metal and organic semiconductors, thereby improving the electron field-effect mobility. Other heterostructures based on peptide-doped cytochrome c can be used to improve the electrical contact in OFETs, in OPV cells, and as connecting units in stacked OPV cells and OLEDs.

The introduction of organic heterostructures has significantly improved device performance and allowed new functions in many applications. For example, the observation of electron-and hole-accumulation layers on both sides of an organic heterojunction suggests that interactions at the heterojunction interface could lead to carrier redistribution and band bending. This ambipolar transport behavior of organic heterojunctions presents the possibility of fabricating OLED FETs with high quantum efficiency. The application of organic heterostructures, including heterostructures formed of peptide-doped cytochrome c, as a buffer layer improve the contact between organic layers and metal electrodes is also discussed. Charge transport in organic semiconductors is influenced by many factors - the present review emphasizes the use of intentionally doped n-and p-type organic semiconductors, and primarily considers organic heterojunctions composed of crystalline organic films displaying band transport behavior.

In general, OFETs operate in accumulation mode. In hole-accumulation mode OFETs, for example, when a negative voltage is applied to the gate relative to the source electrode (which is grounded), the formation of positive charges (holes) is induced in the organic layer near the insulator layer. When the applied gate voltage exceeds the threshold voltage (*V*_{T}), the induced holes form a conducting channel and allow current to flow from the drain to the source under a potential bias (*V*_{DS}) applied to the drain electrode relative to the source electrode. The channel in OFETs contains mobile free holes, and the threshold voltage is the minimum gate voltage required to induce formation of the conducting channel. Therefore, OFETs operate in accumulation mode, or as a 'normally-off' device. However, in some case, OFETs can have an open channel under zero gate voltage, meaning that an opposite gate voltage is required to turn the device off. These devices are therefore called 'normally-on' or 'depletion-mode' transistors.

The charge-carrier type in the conducting channel for the normally-on CuPc/F₁₆CuPc heterojunction transistor is dependent on the bottom-layer semiconductor (organic layer near the insulator). Charge accumulation can lead to upward band bending in the p-type material and downward band bending in n-type material from the bulk to the interface, which is different to the case for a conventional inorganic p-n junction. As free electrons and holes can co-exist in organic heterojunction films, it is possible that organic heterojunction films can transport either electrons or holes, depending on the gate voltage. In fact, after optimizing the film thickness and device configuration, ambipolar transport behavior has been observed.

Carrier transport in planar heterojunction is parallel to the heterojunction interface, similar to the case for OFETs and directly reflecting the conductivity of the heterojunction film. The conductivity of diodes with a double-layer structure can be about one order of magnitude higher than that of single-layer devices, and may be further enhanced by changing the concentration of aromatic-cationic peptide in cytochrome c layers used to form the heterojunction. Suitable ranges of aromatic-cationic peptide concentration include, but are not limited to, 0-500 mM; 0-100 mM; 0-500 µm; 0-250 µm; and 0-100 µm. For the normally-on OFETs, the induced electrons and holes form a conducting channel in the films, leading to high conductivity. Decreased conductivity due the higher roughness of the interface can be compensated by changing the peptide doping concentration as described above.

The induced electrons and holes in n-and p-type semiconductors form a space-charge region at the heterojunction interface, which can result in a built-in electric field from the p-to the n-type semiconductor. Such a build up is revealed in the electronic properties of diodes with vertical structures. A vertical heterojunction diode produces a small current under a positive potential bias and a large current under a negative bias. In contrast with an inorganic p-n diode, an organic heterojunction diode may show a reverse-rectifying characteristic. The positive bias strengthens band bending and restricts carrier flow, whereas under negative bias, the applied electric field opposes the built-in field, resulting in a lowering of the potential barrier. Band bending is therefore weakened under negative bias, and current flow through the junction is assisted.

Charge carrier accumulation on both sides of the organic heterojunction interface creates a built-in field that can be used to shift the threshold voltage of in an OFET. In n-channel organic heterojunction transistors, for example, the threshold voltage is correlated with the trap density in the n-type layer. The induced electrons can fill the traps; therefore, under the conditions of constant n-type layer thickness, the threshold voltage decreases with increasing electron density. Under neutral conditions, the number of induced holes in the p-type layer is equal to that in the n-type layer, and increases with p-type layer thickness tending toward saturation. Therefore, the threshold voltage of organic heterojunction transistors can be reduced by increasing the thickness of the p-type layer. The charge accumulation thickness can be estimated from the point at which the threshold voltage no longer changes with increasing p-type layer thickness.

The difference between the work functions of the two semiconductors constituting a heterojunction leads to various electron states in the space-charge region. The semiconductor heterojunction is also classified by the conductivity type of the two semiconductors forming the heterojunction. If the two semiconductors have the same type of conductivity, then the junction is called an isotype heterojunction; otherwise it is known as anisotype heterojunction. Electrons and holes can be simultaneously accumulated and depleted on both sides of anisotype heterojunctions due to the difference in the Fermi levels of the two components. If the work function of the p-type semiconductor is greater than that of the n-type semiconductor (*ϕ*ₚ *> ϕ*ₙ), depletion layers of electrons and holes are present on either side of the heterojunction, and the space-charge region is composed of immobile negative and positive ions. This type of heterojunction is known as a depletion heterojunction, and most inorganic heterojunctions belong to this class of heterojunction, including the conventional p-n homojunction.

### Cytochrome c Doped with Aromatic-Cationic Peptides for Batteries

Cytochrome c and/or cytochrome c doped with aromatic-cationic peptide, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), can also be used to reduce the internal resistance of batteries, which makes it possible to maintain the battery at nearly constant voltage during discharge. As understood in the art, a battery is a device that converts chemical energy directly to electrical energy. It includes a number of voltaic cells, each of which in turn includes two half cells connected in series by a conductive electrolyte containing anions and cations. One half-cell includes electrolyte and the electrode to which anions (negatively charged ions) migrate, i.e., the anode or negative electrode; the other half-cell includes electrolyte and the electrode to which cations (positively charged ions) migrate, *i.e*., the cathode or positive electrode. In the redox reaction that powers the battery, cations are reduced (electrons are added) at the cathode, while anions are oxidized (electrons are removed) at the anode. The electrodes do not touch each other but are electrically connected by the electrolyte. Some cells use two half-cells with different electrolytes. A separator between half cells allows ions to flow, but prevents mixing of the electrolytes.

Each half cell has an electromotive force (or emf), determined by its ability to drive electric current from the interior to the exterior of the cell. The net emf of the cell is the difference between the emfs of its half-cells. Therefore, if the electrodes have emfs the difference between the reduction potentials of the half-reactions. Peptide-doped cytochrome c can be used to transmit current from interior to the exterior of the cell with a variable or preset conductivity to increase (or decrease) the emf and/or the charging time depending on the application.

The electrical driving force across the terminals of a cell is known as the terminal voltage (difference) and is measured in volts. The terminal voltage of a cell that is neither charging nor discharging is called the open-circuit voltage and equals the emf of the cell. Because of internal resistance, the terminal voltage of a cell that is discharging is smaller in magnitude than the open-circuit voltage and the terminal voltage of a cell that is charging exceeds the open-circuit voltage. An ideal cell has negligible internal resistance, so it would maintain a constant terminal voltage of until exhausted, then dropping to zero. In actual cells, the internal resistance increases under discharge, and the open circuit voltage also decreases under discharge. If the voltage and resistance are plotted against time, the resulting graphs typically are a curve; the shape of the curve varies according to the chemistry and internal arrangement employed. Cytochrome c and/or cytochrome c doped with aromatic-cationic peptide(s) can be used to reduce the internal resistance of the battery in order to provide better performance. For more details on organic batteries, *see, e.g.,* U.S. Patent No. 4,585,717, which is incorporated herein by reference in its entirety.

### Single-Molecule Peptide-Doped Cytochrome c Batteries

Single molecules of cytochrome c can also be used as molecular batteries whose charging and/or discharging time can be regulated by one or more aromatic-cationic peptides, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31). As described herein, cytochrome c is a membrane protein with carbon and sulfur on opposite sides of the membrane from charged oxygen and nitrogen atoms. The regions coated with charged oxygen and nitrogen, which prefer a watery environment, stick out on opposite faces of the membrane. This arrangement is perfect for the job performed by cytochrome c, which uses the reaction of oxygen to water to power a molecular pump. As oxygen is consumed, the energy is stored by pumping hydrogen ions from one side of the membrane to the other. Later, the energy can be used to build ATP or power a motor by letting the hydrogen ions seep back across the membrane.

### Cytochrome c Doped with Aromatic-Cationic Peptides for Photovoltaic (Solar) Cells

Organic photovoltaics (OPV) offers the promise of significant disruption in pricing and aesthetics, as well as impressive efficiencies in low light conditions. OPV materials are also flexible and form-fitting. OPVs can potentially be wrapped around or even painted onto various materials. Current OPV efficiencies are between 5% and 6.25%. Although these efficiencies may not be sufficient to replace conventional forms of power generation, OPV is suitable for applications which do not require significant efficiencies, especially given the high cost of semiconductor solar cells. For example, OPV cells could be used to power cell phones under low light conditions, like those in an office, home or conference room setting, on a continuous trickle-charge setting.

OPV cells, such as those shown in FIGS. 18 and 19, are also cheaper and easier to build than inorganic cells because of simpler processing at much lower temperatures (20-200 °C). For example, electro-chemical solar cells using titanium dioxide in conjunction with an organic dye and a liquid electrolyte already exceeded 6% power conversion efficiencies and are about to enter the commercial market thanks to their relatively low production costs. OPVs can also be processed from solution at room-temperature onto flexible substrates using simple and therefore cheaper deposition methods like spin or blade coating. Possible applications may range from small disposable solar cells to power smart plastic (credit, debit, phone or other) cards which can display for example, the remaining amount, to photo-detectors in large area scanners or medical imaging and solar power applications on uneven surfaces.

An OPV cell (OPVC) is a photovoltaic cell that uses organic electronics, such as cytochrome c and/or cytochrome c doped with an aromatic-cationic peptide, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), for light absorption and charge transport. OPVCs convert visible light into direct current (DC) electricity. Some photovoltaic cells can also convert infrared (IR) or ultraviolet (UV) radiation into DC. The band gap of the active layer (*e.g*., peptide-doped cytochrome c) determines the absorption band of the OPVC.

When these organic band-gap materials absorb a photon, an excited state is created and confined to a molecule or a region of the molecule that absorbs the photon. The excited state can be regarded as an electron hole pair bound together by electrostatic interactions. In photovoltaic cells, excitons are broken up into free electrons-hole pairs by effective fields. The effective field are set up by creating a heterojunction between two dissimilar materials. Effective fields break up excitons by causing the electron to fall from the conduction band of the absorber to the conduction band of the acceptor molecule. It is necessary that the acceptor material has a conduction band edge that is lower than that of the absorber material.

Single-layer OPVCs can be made by sandwiching a layer of organic electronic material (*e.g*., cytochrome c and/or cytochrome c doped with aromatic-cationic peptide(s)) between two metallic conductors, typically a layer of indium tin oxide (ITO) with high work function and a layer of low work function metal such as Al, Mg, or Ca. The difference of work function between the two conductors sets up an electric field in the organic layer. When the organic layer absorbs light, electrons will be excited to the conduction band and leave holes in the valence band, forming excitons. The potential created by the different work functions helps to separate the exciton pairs, pulling electrons to the cathode and holes to the anode. The current and voltage resulting from this process can be used to do work.

In practice, single-layer OPVCs have low quantum efficiencies (<1%) and low power conversion efficiencies (<0.1%). A major problem with them is the electric field resulting from the difference between the two conductive electrodes is seldom sufficient to break up the photo-generated excitons. Often the electrons recombine with the holes rather than reach the electrode.

Organic heterojunctions can be used to make built-in fields for enhancing OPVC performance. Heterojunctions are implemented by incorporating two or more different layers in between the conductive electrodes. These two or more layers of materials have differences in electron affinity and ionization energy, *e.g.,* due to peptide concentration, that induce electrostatic forces at the interface between the two layers. The materials are chosen properly to make the differences large enough, so these local electric fields are strong, which may break up the excitons much more efficiently than the single layer photovoltaic cells do. The layer with higher electron affinity (*e.g*., higher peptide doping concentration) and ionization potential is the electron acceptor, and the other layer is the electron donor. This structure is also called planar donor-acceptor heterojunctions.

The electron donor and acceptor can be mixed together to form a bulk heterojunction OPVC. If the length scale of the blended donor and acceptor is similar with the exciton diffusion length, most of the excitons generated in either material may reach the interface, where excitons break efficiently. Electrons move to the acceptor domains then were carried through the device and collected by one electrode, and holes were pulled in the opposite direction and collected at the other side.

Difficulties associated with organic photovoltaic cells include their low quantum efficiency (∼3%) in comparison with inorganic photovoltaic devices; due largely to the large band gap of organic materials. Instabilities against oxidation and reduction, recrystallization and temperature variations can also lead to device degradation and decreased performance over time. This occurs to different extents for devices with different compositions, and is an area into which active research is taking place. Other important factors include the exciton diffusion length; charge separation and charge collection; and charge transport and mobility, which are affected by the presence of impurities. For more details on organic photovoltaics, *see, e.g.,* U.S. Patent No. 6,657,378; U.S. Patent No. 7,601,910; and U.S. Patent No. 7,781,670, each of which is herein incorporated by reference in its entirety.

### Thin-Film Applications of Cytochrome c Doped with Exemplary Aromatic-Cationic Peptides

As well understood by those of ordinary skill in the art of electronic, any of the aforementioned devices can be made by depositing, growing, or otherwise providing thin layers of material to form an appropriate structure. For example, heterojunctions for transistors, diodes, and photovoltaic cells can be formed by depositing layers of material with different band gap energies adjacent to each other or in layered fashion. In addition to forming layered thin-film structures, organic materials with different band gaps can be mixed to form heterojunctions with varied spatial arrangements, as shown in FIGS. 19(a) and 19(b), by depositing heterogeneous mixtures of material. Such heterogeneous mixtures may include, but are not limited to, mixtures of cytochrome c, aromatic-cationic peptides and cytochrome c doped with varying levels of aromatic-cationic peptides, including, but not limited to such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31). Illustrative aromatic-cationic peptide levels may include, but are not limited to, 0-500 mM; 0-100 mM; 0-500 µM; 0-250 µM; and 0-100 µM. These thin films may also be used to enhance performance of conventional electronic devices, *e.g*., by increasing conductivity and/or reducing heat dissipation at electrodes.

As described above, dispersed hetero-junction of donor-acceptor organic materials have high quantum efficiency compared to the planar hetero-junction, because it is more likely for an exciton to find an interface within its diffusion length. Film morphology can also have a drastic effect on the quantum efficiency of the device. Rough surfaces and presence of voids can increase the series resistance and also the chance of short circuiting. Film morphology and quantum efficiency can be improved by annealing of a device after covering it by with a metal cathode having a thickness of about 1000 Å. Metal film on top of the organic film applies stresses on the organic film, which helps to prevent the morphological relaxation in the organic film. This gives more densely packed films while at the same time allows the formation of phase-separated interpenetrating donor-acceptor interface inside the bulk of organic thin film.

Controlled growth of the heterojunction provides better control over positions of the donor-acceptor materials, resulting in much greater power efficiency (ratio of output power to input power) than that of planar and highly disoriented hetero-junctions. This is because charge separation occurs at the donor acceptor interface: as the charge travels to the electrode, it can become trapped and/or recombine in a disordered interpenetrating organic material, resulting in decreased device efficiency. Choosing suitable processing parameters to better control the structure and film morphology mitigates undesired premature trapping and/or recombination.

### Depositing Cytochrome c Doped with Aromatic-Cationic Peptides

Organic films including cytochrome c, an aromatic-cationic peptide, or cytochrome c doped with aromatic-cationic peptide, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), for photovoltaic and other applications maybe deposited by spin coating, vapor-phase deposition, and method described in U.S. Patent No. 6,734,038; U.S. Patent No. 7,662,427; and U.S. Patent No. 7,799,377, each of which is incorporated herein by reference in its entirety. Spin-coating techniques can be used to coat larger surface areas with high speed but the use of solvent for one layer can degrade the any already existing polymer layers. Spin-coated materials must be patterned in a separate patterning step.

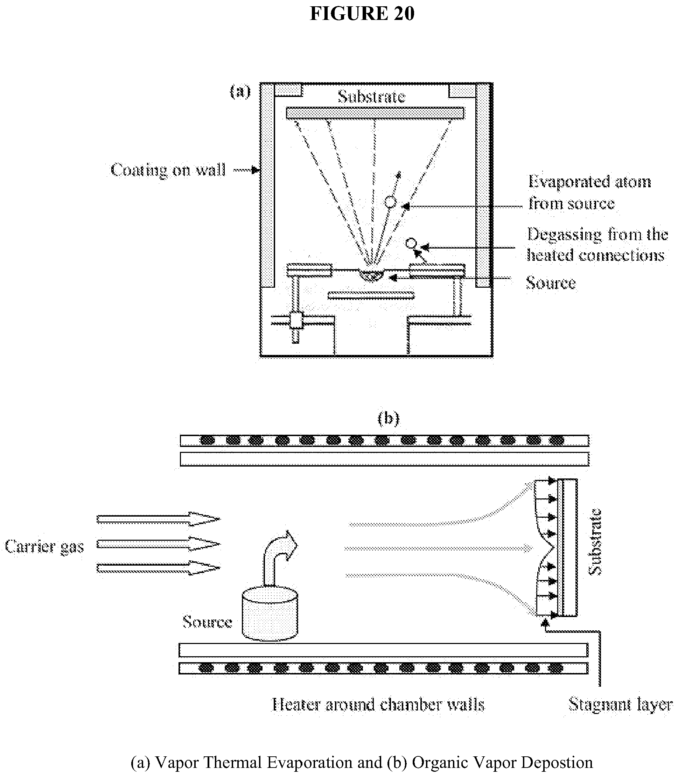

Vacuum thermal evaporation (VTE), as shown in FIG. 20(a), is a deposition technique that involves heating the organic material in vacuum. The substrate is placed several centimeters away from the source so that evaporated material may be directly deposited onto the substrate. VTE is useful for depositing many layers of different materials without chemical interaction between different layers.

Organic vapor phase deposition (OVPD), as shown in FIG. 20(b), gives better control on the structure and morphology of the film than vacuum thermal evaporation. OPVD involves evaporation of the organic material over a substrate in the presence of an inert carrier gas. The morphology of the resulting film can be changed by changing the gas flow rate and the source temperature. A uniform film can be grown by reducing the carrier gas pressure, which increases the velocity and mean free path of the gas, which results in a decrease of the boundary layer thickness. Cells produced by OVPD do not have issues related with contaminations from the flakes coming out of the walls of the chamber, as the walls are warm and do not allow molecules to stick to and produce a film upon them. Depending on the growth parameters (*e.g*., temperature of the source, base pressure and flux of the carrier gas, *etc*.) the deposited film can be crystalline or amorphous in nature. Devices fabricated using OVPD show a higher short-circuit current density than that of devices made using VTE. An extra layer of donor-acceptor hetero-junction at the top of the cell may block excitons, while allowing conduction of electron, resulting in improved cell efficiency.

### Cytochrome c Doped with Exemplary Aromatic-Cationic Peptides for Increasing Efficiency

As described above, exemplary aromatic-cationic peptides, such as Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31), can be used to increase conductivity. As a result, exemplary aromatic-cationic peptides can be used to conduct electric current with lower loss through the production of (waste) heat energy. This effect can be exploited to extend the operating life of battery-powered devices, such as consumer electronics, and in large power systems, such as in power transmission applications. The reduction of waste heat production also lowers cooling requirements, further increasing efficiency, and extends the lifetime of electronic devices powered by conductive materials, such as cytochrome c, doped with aromatic-cationic peptides of the invention.

### Aromatic-cationic peptides for cytochrome c biosensor applications

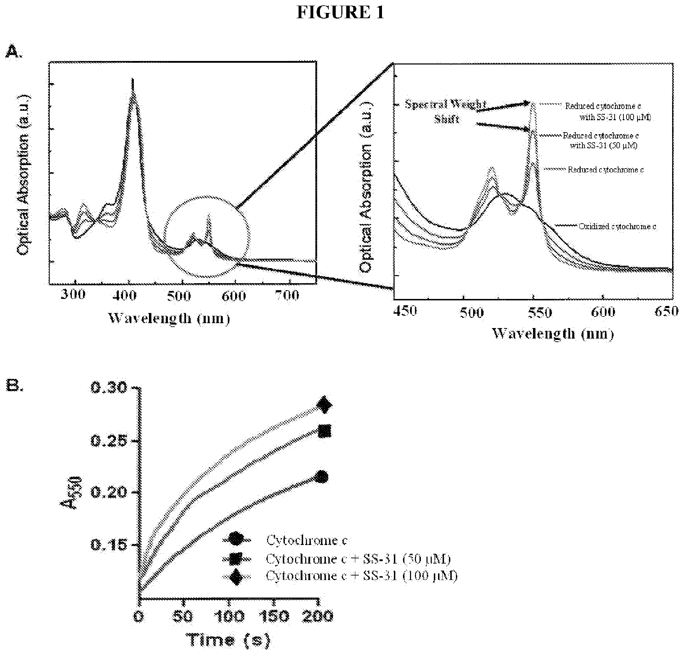

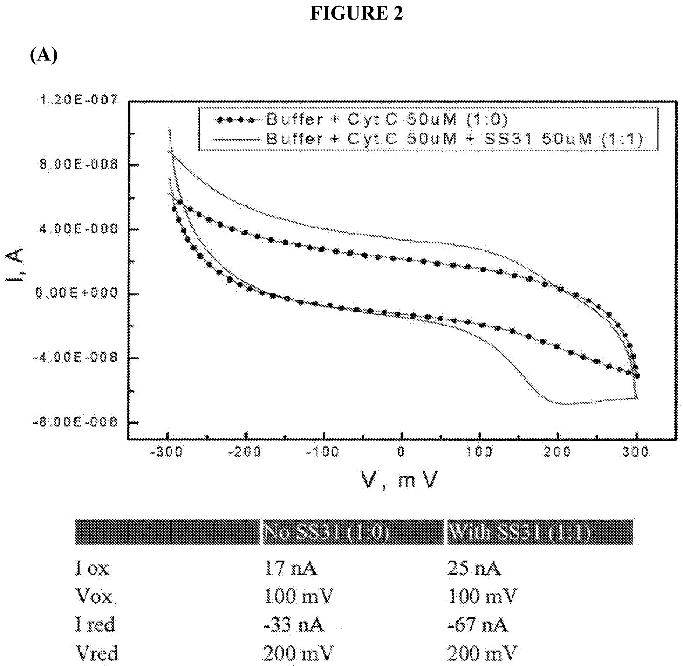

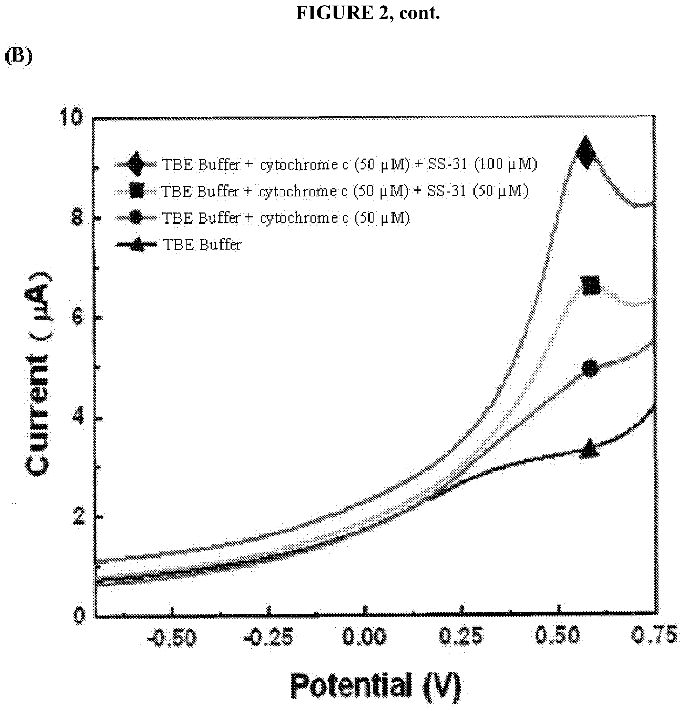

The aromatic-cationic peptides disclosed herein may be used to enhance electron flow in cytochrome c biosensors and to increase their levels of sensitivity. As illustrated by the examples, the peptides disclosed herein, such as the peptide D-Arg-2',6'-Dmt-Lys-Phe-NH₂, promote the reduction of cytochrome c (FIG. 1) and increase electron flow through cytochrome c (FIG. 2).

Cytochrome c is a promising biosensor candidate from an electrochemical viewpoint. However, electron transfer between heme and a bare electrode is usually slow. Alternatively, small mediators may be used to facilitate electron transfer between the redox-active center and the electrode indirectly. Additionally or alternatively, direct electron transfer methods may be used whereby redox-active enzyme are immobilized directly onto the electrode surface. For example, cytochrome c, which is positively charged at pH 7 and contains a large number of Lys residues surrounding the heme edge, adsorbs on negatively charged surfaces created, for example, by self-assembling carboxy terminated alkanethiols. In some embodiments, at a constant potential of +150 mV, the cytochrome c electrode is sensitive to superoxide in the nM concentration range.

In some aspects, the present disclosure provides methods and compositions for increasing the sensitivity of cytochrome c biosensors. In some embodiments, the cytochrome c biosensor includes one or more of the aromatic-cationic peptides disclosed herein. In some embodiments, peptide-doped cytochrome c serves as a mediator between a redox-active enzyme and an electrode within the biosensor. In some embodiments, peptide-doped cytochrome c is immobilized directly on the electrode of the biosensor. In some embodiments, the peptide is linked to cytochrome c within the biosensor. In other embodiments, the peptide is not linked to cytochrome c. In some embodiments, the peptide and/or cytochrome c are immobilized on a surface within the biosensor. In other embodiments, the peptide and/or cytochrome c are freely diffusible within the biosensor. In some embodiments, the biosensor includes the peptide D-Arg-2',6'-Dmt-Lys-Phe-NH₂ and/or Phe-D-Arg-Phe-Lys-NH₂.

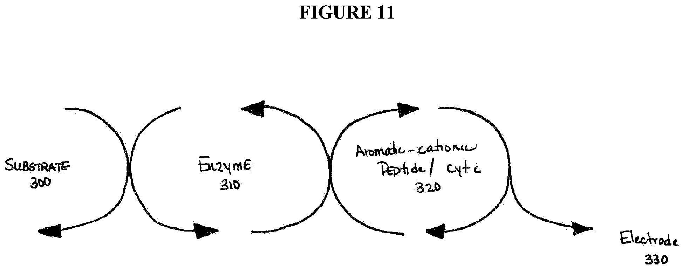

Figure 11 shows electron flow within a biosensor in which aromatic-cationic peptides and cytochrome c serve as mediators of electron flow from a redox-active enzyme to an electrode. In serial redox reactions, electrons are transferred from a substrate 300 to a redox-active enzyme 310, from the enzyme 310 to peptide-doped cytochrome c 320, and from peptide-doped cytochrome c 320 to an electrode 330.

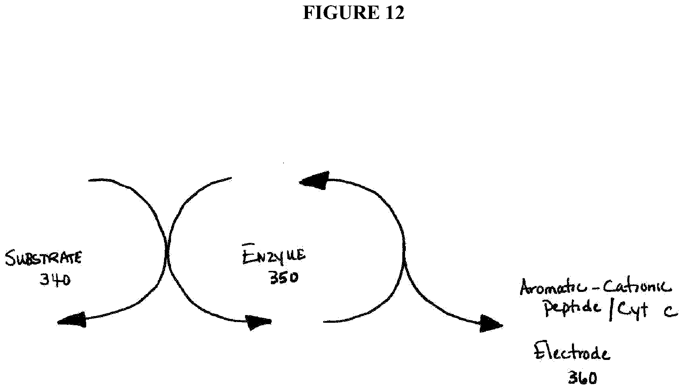

Figure 12 shows electron flow within a biosensor in which aromatic-cationic peptides and cytochrome c are immobilized directly on the electrode. In serial redox reactions, electrons are transferred from a substrate 340 to a redox-active enzyme 350, and from the enzyme 350 to an electrode 360 on which peptide-doped cytochrome c is immobilized.

### Aromatic-cationic Peptides in Bioremediation of Environmental Contaminants

The aromatic-cationic peptides disclosed herein are useful for the bioremediation of environmental contaminants. In particular, the peptides are useful for increasing the rate and/or efficiency of bioremediation reactions in which bacterial c cytochromes mediate the transfer of electrons to an environmental contaminant, thereby altering the valence of the substance and reducing its relative toxicity. In the methods disclosed herein, aromatic-cationic peptides interact with bacterial c cytochromes and facilitate electron transport. In one aspect, the aromatic-cationic peptides facilitate reduction of bacterial c cytochromes. In another aspect, the peptides enhance electron diffusion through bacterial c cytochromes. In another aspect, the peptides enhance electron capacity in bacterial c cytochromes. In another aspect, the peptides induce novel π-π interactions around the heme groups of bacterial cytochromes that favor electron diffusion. Ultimately, interaction of the aromatic-cationic peptides with bacterial c cytochromes promotes and/or enhances the dissimilatory reduction of the environmental contaminant.

In one aspect, the present disclosure provides methods and compositions for the bioremediation of environmental contaminants. In general, the methods comprise contacting a sample that contains an environmental contaminant with a bioremedial composition under conditions conducive to dissimilatory reduction of the particular contaminant present in the sample. In general, the bioremedial composition comprises recombinant bacteria expressing one or more of the aromatic-cationic peptides disclosed herein.

In some embodiments, the bioremedial compositions described herein comprise recombinant bacteria that express one or more aromatic-cationic peptides disclosed herein from an exogenous nucleic acid. In some embodiments, the nucleic acid encodes the peptide. In some embodiments, the nucleic acid encoding the peptide is carried on a plasmid DNA that is taken up by the bacteria through bacterial transformation. Examples of bacterial expression plasmids that may be used in the methods described herein include but are not limited to ColE1, pACYC184, pACYC177, pBR325, pBR322, pUC118, pUC119, RSF1010, R1162, R300B, RK2, pDSK509, pDSK519, and pRK415.

In some embodiments, the bioremedial composition comprises recombinant bacteria that express aromatic-cationic peptides disclosed herein from a stable genomic insertion. In some embodiments, the genomic insertion comprises a nucleic acid sequence that encodes the peptide. In some embodiments, the nucleic acid sequence is carried by a bacterial transposon that integrates into the bacterial genome. Examples of bacterial transposons that may be used in the methods described herein include but are not limited to Tn1, Tn2, Tn3, Tn21, gamma delta (Tn1000), Tn501, Tn551, Tn801, Tn917, Tn1721 Tn1722 Tn2301.

In some embodiments, nucleic acid sequences encoding aromatic-cationic peptides are under the control of a bacterial promoter. In some embodiments, the promoter comprises an inducible promoter. Examples of inducible promoters that may be used in the methods described herein include but are not limited to heat-shock promoters, isopropyl β-D-1-thiogalactopyranoside (IPTG)-inducible promoters, and tetracycline (Tet)-inducible promoters.

In some embodiments, the promoter comprises a constitutive promoter. Examples of constitutive promoters that may be used in the methods described herein include but are not limited to the spc ribosomal protein operon promoter (Pspc), the beta-lactamase gene promoter (Pbla), the PL promoter of lambda phage, the replication control promoters PRNAI and PRNAII, and the P1 and P2 promoters of the rmB ribosomal RNA operon.

In some embodiments, the recombinant bacteria comprises the genus Shewenella. In some embodiments, the bacteria comprises *S. abyssi, S. algae, S. algidipiscicola, S. amazonensis, S. aquimarina, S. baltica, S. benthica, S. colwelliana, S. decolorationis, S. denitrificans, S. donghaensis, S. fidelis, S. ftigidimarina, S. gaetbuli, S. gelidimarina, S. glacialipiscicola, S. hafniensis, S. halifaxensis, S. hanedai, S. irciniae, S. japonica, S. kaireitica, S. livingstonensis, S. loihica, S. marinintestina, S. marisflavi, S. morhuae, S. olleyana, S. oneidensis,S. pacifica, S. pealeana, S. piezotolerans, S. pneumatophori, S. profunda, S. psychrophila, S. putrefaciens, S. sairae, S. schegeliana, S. sediminis, S. spongiae, S. surugensis, S. violacea, S. waksmanii,* or *S. woodyi.*

In some embodiments, the recombinant bacteria comprises the genus Geobacter. In some embodiments, the bacteria comprises *G. ferrireducens, G. chapellei, G. humireducens, G. arculus, G. sullfurreducens, G. hydrogenophilus, G. metallireducens, G. argillaceus, G. bemidjiensis, G. bremensis, G. grbiciae, G. pelophilus, G. pickeringii, G. thiogenes, or G. uraniireducens.*

In some embodiments, the recombinant bacteria comprises the genus Desulfuromonas. In some embodiments, the bacteria comprises *D. palmitatis, D. chloroethenica, D. acetexigens, D. acetoxidans, D. michiganensis,* or *D. thiophila, D. sp.*

In some embodiments, the recombinant bacteria comprises the genus Desulfovibrio. In some embodiments, the bacteria comprises *Desulfovibrio africanus, Desulfovibrio baculatus, Desulfovibrio desulfuricans, Desulfovibrio gigas, Desulfovibrio halophilus, Desulfovibrio magneticus, Desufovibrio multispirans, Desulfovibrio pigra, Desulfovibrio salixigens, Desulfovibrio sp.,* or *Desulfovibrio vulgaris.*

In some embodiments, the recombinant bacteria comprises the genus Desulfuromusa. In some embodiments, the bacteria comprises *D. bakii, D. kysingii,* or *D. succinoxidans.*

In some embodiments, the recombinant bacteria comprises the genus Pelobacter. In some embodiments, the bacteria comprises *P. propionisus, P. acetylinicus, P. venetianus, P. carbinolicus, P. cidigallici, P. sp. A3b3, P. masseliensis, or P. seleniigenes.*

In some embodiments, the recombinant bacteria comprises *Thermotoga maritima, Thermoterrobacterium ferrireducens, Deferribacter thermophilus, Geovibrio ferrireducens, Desulfobacter propionicus, Geospirillium barnseii, Ferribacterium limneticum, Geothrix fermentens, Bacillus infernus, Thermas sp. SA-01, Escherichia coli, Proteus mirabilis, Rhodobacter capsulatus, Rhodobacter sphaeroides, Thiobacillus denitrificans, Micrococcus denitrificans, Paraoccus denitrificans,* or *Pseudomonas sp.*

In some embodiments, the methods disclosed herein relate to the dissimilatory reduction of a metal. In some embodiments, the metal comprises Sc, Ti, V, Cr, Mn, Fe, Co, Ni, Cu, Zn, Y, Zr, Nb, Mo, Tc, Ru, Pd, Ag, Cd, Hf, Ta, W, Re, Os, Ir, Pt, Au, Hg, Rf, Db, Sg, Bh, Hs, Cn, Al, Ga, In, Sn, Ti, Pb, or Bi.. In some embodiments, the methods result in the formation of an insoluble oxide. In some embodiments, the methods result in the reduction of Cr(VI) to Cr(III) and the formation of an insoluble precipitate. In some embodiments, methods for metal bioremediation comprise contacting the metal with a bioremedial composition comprising bacteria listed in Table 7 engineered to express one or more aromatic-cationic peptides disclosed herein.

In some embodiments, the methods disclosed herein relate to the dissimilatory reduction of a non-metal. In some embodiments, the non-metal comprises sulfate. In some embodiments, the methods result in the reduction of sulfate and the formation of hydrogen sulfide. In some embodiments, sulfate bioremediation methods comprise contacting the sulfate with a bioremedial composition comprising bacteria listed in Table 7 engineered to express one or more aromatic-cationic peptides disclosed herein.

In some embodiments, the methods disclosed herein relate to the dissimilatory reduction of a perchlorate. In some embodiments, the perchlorate comprises, NH₄ClO₄, CsClO₄, LiClO₄, Mg(ClO₄)₂, HClO₄, KClO₄, RbClO₄, AgClO₄, or NaClO₄. In some embodiments, the methods result in the reduction of perclorates to chlorites. In some embodiments, perchlorate bioremediation methods comprise contacting perchlorates with a bioremedial composition comprising *E. coli, Proteus mirabilis, Rhodobacter capsulatus,* or *Rhodobacter sphaeroides* engineered to express one or more aromatic-cationic peptides disclosed herein. In some embodiments, perchlorate bioremediation methods comprise contacting perchlorate with a bioremedial composition comprising bacteria listed in Table 7 engineered to express one or more aromatic-cationic peptides disclosed herein.

In some embodiments, the methods disclosed herein relate to the dissimilatory reduction of a nitrate. In some embodiments, the nitrate comprises HNO₃, LiNO₃, NaNO₃, KNO₃, RbNO₃, CsNO₃, Be(NO₃)₂, Mg(NO₃)₂, Ca(NO₃)₂, Sr(NO₃)₂, Ba(NO₃)₂, Sc(NO₃)₃, Cr(NO₃)₃, Mn(NO₃)₂, Fe(NO₃)₃, Co(NO₃)₂, Ni(NO₃)₂, Cu(NO₃)₂, Zn(NO₃)₂, Pd(NO₃)₂, Cd(NO₃)₂, Hg(NO₃)₂, Pb(NO₃)₂, or Al(NO₃)₃. In some embodiments, the methods result in the reduction of nitrates to nitrites. In some embodiments, nitrate bioremediation methods comprise contacting nitrates with a bioremedial composition comprising *Thiobacillus denitrificans, Micrococcus denitrificans, Paraoccus denitrificans, Pseudomonas sp.,* or *E. coli* engineered to express one or more aromatic-cationic peptides disclosed herein. In some embodiments, nitrate bioremediation methods comprise contacting the nitrate with a bioremedial composition comprising bacteria listed in Table 7 engineered to express one or more aromatic-cationic peptides disclosed herein.

| **Table 7. Illustrative Bioremedial Bacterial species.** | | |
|---|---|---|
| *Shewenella abyssi* | *Shewenella sairae* | *Desulfuromonas chloroethenica* |
| *Shewenella algae* | *Shewenella schegeliana* | *Desulfuromonas acetexigens* |
| *Shewenella algidipiscicola* | *Shewenella sediminis* | *Desulfuromonas acetoxidans* |
| *Shewenella amazonensis* | *Shewenella spongiae* | *Desulfuromonas michiganensis* |
| *Shewenella aquimarina* | *Shewenella surugensis* | *Desulfuromonas thiophila* |
| *Shewenella baltica* | *Shewenella violacea* | *Desulfuromonas sp.* |
| *Shewenella benthica* | *Shewenella waksmanii* | *Desulfuromusa bakii* |
| *Shewenella colwelliana* | *Shewenella woodyi* | *Desulfuromusa kysingii* |
| *Shewenella decolorationis* | *Desulfovibrio africanus* | *Desulfuromusa succinoxidans* |
| *Shewenella denitrificans* | *Desulfovibrio baculatus* | *Pelobacter propionisus* |
| *Shewenella donghaensis* | *Desulfovibrio desulfuricans* | *Pelobacter acetylinicus* |
| *Shewenella fidelis* | *Desulfovibrio gigas* | *Pelobacter venetianus* |
| *Shewenella frigidimarina* | *Desulfovibrio halophilus* | *Pelobacter arbinolicus* |
| *Shewenella gaetbuli* | *Desulfovibrio magneticus* | *Pelobacter acidigallici* |
| *Shewenella gelidimarina* | *Desulfovibrio multispirans* | *Pelobacter sp. A3b3* |
| *Shewenella glacialipiscicola* | *Desulfovibrio pigra* | *Pelobacter masseliensis* |
| *Shewenella hafniensis* | *Desulfovibrio salixigens* | *Pelobacter seleniigenes* |
| *Shewenella halifaxensis* | *Desulfovibrio sp.* | *Thermotoga maritime* |
| *Shewenella hanedai* | *Desulfovibrio vulgaris* | *Thermoterrobacterium* |
| *Shewenella irciniae* | *Geobacter ferrireducens* | *ferrireducens* |
| *Shewenella japonica* | *Geobacter chapellei* | *Deferribacter thermophilus* |
| *Shewenella kaireitica* | *Geobacter humireducens* | *Geovibrio ferrireducens* |
| *Shewenella livingstonensis* | *Geobacter arculus* | *Desulfobacter propionicus* |
| *Shewenella loihica* | *Geobacter sullfurreducens* | *Geospirillium barnseii* |
| *Shewenella marinintestina* | *Geobacter hydrogenophilus* | *Ferribacterium limneticum* |
| *Shewenella marisflavi* | *Geobacter metallireducens* | *Geothrix fermentens* |
| *Shewenella morhuae* | *Geobacter argillaceus* | *Bacillus infernus* |
| *Shewenella olleyana* | *Geobacter bemidjiensis* | *Thermas sp. SA-01* |
| *Shewenella oneidensis* | *Geobacter bremensis* | *Escherichia coli* |
| *Shewenella pacifica* | *Geobacter grbiciae* | *Proteus mirabilis* |
| *Shewenella pealeana* | *Geobacter pelophilus* | *Rhodobacter capsulatus* |
| *Shewenella piezotolerans* | *Geobacter pickeringii* | *Rhodobacter sphaeroides* |
| *Shewenella pneumatophori* | *Geobacter thiogenes* | *Thiobacillus denitrificans* |
| *Shewenella profunda* | *Geobacter uraniireducens* | *Micrococcus denitrificans* |
| *Shewenella psychrophila* | *Desulfuromonas palmitatis* | *Paraoccus denitrificans* |
| *Shewenella putrefaciens* | | *Pseudomonas sp.* |

In some embodiments, the methods disclosed herein relate to the dissimilatory reduction of a radionuclide. In some embodiments, the radionuclide comprises an actinide. In some embodiments, the radionuclide comprises uranium (U). In some embodiments, the methods result in the reduction of U(VI) to U(IV) and the formation of an insoluble precipitate. In some embodiments, the methods relate to the dissimilatory reduction of methyl-tert-butyl ether (MTBE), vinyl chloride, or dichloroethylene. In some embodiments, the bioremediation methods comprise contacting these contaminants with a bioremedial composition comprising bacteria listed in Table 7 engineered to express one or more aromatic-cationic peptides disclosed herein.

In some embodiments, the methods disclosed herein comprise *in situ* bioremediation, wherein a bioremedial composition described herein is administered at the site of environmental contamination. In some embodiments, the methods comprise *ex situ* bioremediation, wherein contaminated materials are removed from their original location and treated elsewhere.

In some embodiments, *ex situ* bioremediation comprises landfarming, wherein contaminated soil is excavated from its original location, combined with a bioremedial composition described herein, spread over a prepared bed, and regularly tilled until the contaminants are removed or reduced to acceptable levels. In some embodiments, *ex situ* bioremediation comprises composting, wherein contaminated soil is excavated from its original location, combined with a bioremedial composition described herein and non-hazardous organic materials, and maintained in a composting container until the contaminants are removed or reduced to acceptable levels. In some embodiments, *ex situ* bioremediation comprises decontamination in a bioreactor, wherein contaminated soil or water is placed in an engineered containment system, mixed with a bioremedial composition described herein, and maintained until the contaminants are removed or reduced to acceptable levels.

Methods for generating recombinant bacteria described herein are well known in the art. The skilled artisan will understand that a number of conventional molecular biology techniques may be used to generate bacterial plasmids encoding one or more aromatic-cationic peptides. For example, nucleic acid sequences encoding the peptides may be synthesized and cloned into the plasmid of choice using restriction and ligation enzymes. Ligation products may be transformed into *E. coli* in order to generate large quantities of the product, which may then be transformed into the bioremedial bacteria of choice. Similarly, strategies may be used to generate bacterial transposons that carry nucleic acid sequencea encoding one or more aromatic-cationic peptides, and to transform the transposon in to the bioremedial bacteria of choice.

The skilled artisan will also understand that routine methods of bacteriology may be used to generate large quantities of recombinant bacteria described herein for use in large-scale bioremediation operations. The skilled artisan will understand that the precise culture conditions will vary depending on the particular bacterial species in use, and that culturing conditions for various bioremedial bacterial are readily available in the art.

General references for bioremediation and other related applications are provided in the following references, which are hereby incorporated by reference in their entirety: U.S. Patent No. 6,913,854; Reimers, C.E. et al. "Harvesting Energy from Marine Sediment-Water Interface" Environ. Sci. Technol. 2001, 35,192-195, Nov. 16, 2000; Bond D.R. et al. "Electrode Reducing Microorgaisms that Harvest Energy from Marine Sediments" Science, vol. 295, 483-485 Jan. 18, 2002; Tender, L.M. et al. "Harnessing Microbially Generated Power on the Seafloor" Nature Biology, vol. 20, pp. 821-825, Aug. 2002; DeLong, E.F. et al. "Power From the Deep" Nature Biology, vol. 20, pp. 788-789, Aug. 2002; Bilal, "Thermo-Electrochemical Reduction of Sulfate to Sulfide Using a Graphite Cathode," J. Appl. Electrochem., 28, 1073, (1998); Habermann, et al., "Biological Fuel Cells With Sulphide Storage Capacity," Applied Microbiology Biotechnology, 35, 128, (1991); and Zhang, et al., "Modelling of a Microbial Fuel Cell Process," Biotechnology Letters, vol. 17 No. 8, pp. 809-814 (Aug., 1995).

### Aromatic-cationic Peptides and cytochrome c in nanowire applications

The aromatic-cationic peptides disclosed herein, cytochrome c, and/or peptide doped cytochrome c are useful in nonowire applications. Typically, a nanowire is a nanostructure, with the diameter of the order of a nanometer (10⁻⁹ meters). Alternatively, nanowires can be defined as structures that have a thickness or diameter constrained to tens of nanometers or less and an unconstrained length. At these scales, quantum mechanical effects come into play. Many different types of nanowires exist, including metallic (e.g., Ni, Pt, Au), semiconducting (e.g., Si, InP, GaN, etc.), and insulating (e.g., SiO2, TiO2). Molecular nanowires are composed of repeating molecular units either organic (e.g. DNA, aromatic-cationic peptides disclosed herein, cytochrome c, and/or peptide doped cytochrome c, etc.) or inorganic (e.g. Mo6S9-xIx). The nanowires disclosed herein are useful, for example, to link components into extremely small circuits. Using nanotechnology, the components are created out of chemical compounds.

### Nanowire synthesis

There are two basic approaches of synthesizing nanowires: top-down and bottom-up approach. In a top-down approach a large piece of material is cut down to small pieces through different means such as lithography and electrophoresis. Whereas in a bottom-up approach the nanowire is synthesized by the combination of constituent ad-atoms. Most of the synthesis techniques are based on bottom-up approach.

Nanowire structures are grown through several common laboratory techniques including suspension, deposition (electrochemical or otherwise), and VLS growth.

A suspended nanowire is a wire produced in a high-vacuum chamber held at the longitudinal extremities. Suspended nanowires can be produced by: the chemical etching, or bombardment (typically with highly energetic ions) of a larger wire; indenting the tip of a STM in the surface of a metal near its melting point, and then retracting it.

Another common technique for creating a nanowire is the Vapor-Liquid-Solid (VLS) synthesis method. This technique uses as source material either laser ablated particles or a feed gas (such as silane). The source is then exposed to a catalyst. For nanowires, the best catalysts are liquid metal (such as gold) nanoclusters, which can either be purchased in colloidal form and deposited on a substrate or self-assembled from a thin film by dewetting. This process can often produce crystalline nanowires in the case of semiconductor materials. The source enters these nanoclusters and begins to saturate it. Once supersaturation is reached, the source solidifies and grows outward from the nanocluster. The final product's length can be adjusted by simply turning off the source. Compound nanowires with super-lattices of alternating materials can be created by switching sources while still in the growth phase. In some embodoments, source material such as aromatic-cationic peptides, cytochrome c and/or peptide doped cytochrome c may be used. Inorganic nanowires such as Mo6S9-xIx (which are alternatively viewed as cluster polymers) are synthesised in a single-step vapour phase reaction at elevated temperature.

In addition, nanowires of many types of materials, such as aromatic-cationic peptides, cytochrome c and/or peptide doped cytochrome c, can be grown in solution. Solution-phase synthesis has the advantage that it can be scaled-up to produce very large quantities of nanowires as compared to methods that produce nanowires on a surface. The polyol synthesis, in which ethylene glycol is both solvent and reducing agent, has proven particularly versatile at producing nanowires of Pb, Pt, and silver.

### General Methods

*Cytochrome c reduction*: increasing amounts of aromatic-cationic peptides were added to a solution of oxidized cytochrome c. The formation of reduced cytochrome c was monitored by absorbance at 500 nm. The rate of cytochrome c reduction was determined by non-linear analysis (Prizm software).

Time-resolved UV-Visible absorption spectroscopy was used to study the electron transport process of cytochrome c in the presence of peptides. Reduced cytochrome c was monitored by absorbance at a broad-band spectral range (200-1100 nm). The absorption changes were recorded with a UV/Visible spectrophotometer (Ultrospec 3300 pro, GE) in quartz cells with path lengths of 1 or 2 mm. N-acetylcysteine (NAC) and glutathione were used as electron donors to reduce oxidized cytochrome c. The rate constant of cytochrome c reduction was estimated by adding various concentrations of peptides. The dose dependence of the peptides was correlated to the cytochrome c reduction kinetics.

*Mitochondrial O₂ consumption and ATP production*: Fresh mitochondria were isolated from rat kidney as described previously. Electron flux was measured by O₂ consumption (Oxygraph Clark electrode) as previously described using different substrates for C1 (glutamate/malate), C2 (succinate), and C3 (TMPD/ascorbate). Assays were carried out under low substrate conditions in order to avoid saturating the enzyme reactions. ATP production in isolated mitochondria was determined kinetically using the luciferase method (Biotherma) in a 96-well luminescence plate reader (Molecular Devices). The initial maximal rate for ATP synthesis was determined over the first minute.

*Cyclic voltammetry*: Cyclic voltammetry was performed using the Bioanalytical System CV-50W Voltammetric Analyzer using an Ag/AgCl/1 M KCl reference electrode with a potential of +0.237 V versus NHE (Biometra, Göttingen, Germany), and a platinum counter electrode. Gold wire electrodes were cleaned following an established protocols. Electrochemical studies of cytochrome c in solution were performed using mercaptopropanol-modified electrodes (incubation 24 h in 20 mM mercaptopropanol). Cyclic voltammograms with 20 µM cytochrome c in 1 M KCl and 10 mM sodium phosphate buffer, pH 7.4 /7.8 were recorded. The formal potential was calculated as the midpoint between the anodic and cathodic peak potentials at different scan rates (100-400 mV/s) and diffusion coefficients from the peak currents at different scan rates according the Randles-Sevcik equation.

### EXAMPLES

The present invention is further illustrated by the following examples, which should not be construed as limiting in any way.

### Example 1. Synthesis of Aromatic-Cationic Peptides.

Solid-phase peptide synthesis is used and all amino acids derivatives are commercially available. After completion of peptide assembly, peptides are cleaved from the resin in the usual manner. Crude peptides are purified by preparative reversed-phase chromatography. The structural identity of the peptides is confirmed by FAB mass spectrometry and their purity is assessed by analytical reversed-phase HPLC and by thin-layer chromatography in three different systems. Purity of >98% will be achieved. Typically, a synthetic run using 5 g of resin yields about 2.0-2.3 g of pure peptides.

### Example 2. The peptide D-Arg-2',6'-Dmt-Lys-Phe-NH₂ (SS-31) facilitates cytochrome c reduction.

Absorption spectroscopy (UltroSpec 3300 Pro; 220-1100 nm) was used to determine if SS-31 modulates cytochrome c reduction (FIG. 1). Reduction of cytochrome c with glutathione is associated with multiple shifts in the Q band (450-650 nm), with a prominent shift at 550 nm. Addition of SS-31 produced significant spectral weight shift at 550 nm (FIG. 1A). Time-dependent spectroscopy show that SS-31 increased the rate of cytochrome c reduction (FIG. 1B). These data show that SS-31 altered the electronic structure of cytochrome c and enhanced the reduction of Fe3+ to Fe2+ heme.

### Example 3. The peptide D-Arg-2',6'-Dmt-Lys-Phe-NH2 (SS-31) enhances electron diffusion through cytochrome c.

Cyclic voltammetry (CV) was carried out to determine if SS-31 altered electron flow and/or reduction/oxidation potentials of cytochrome c (FIG. 2, upper panel). CV was done using an Au working electrode, Ag/AgCl reference electrode, and Pt auxiliary electrode. SS-31 increased current for both reduction and oxidation processes of cytochrome c (FIG. 2, upper panel). SS-31 does not alter reduction/oxidation potentials (FIG. 2, upper panel), but rather increases electron flow through cytochrome c, demonstrating that SS-31 decreases resistance between complexes III to IV. For FIG 2 (lower panel) all voltammetric measurements were performed using the BASi-50W Voltammetric Analyzer coupled to a BASi C3 Cell Stand. An Ag/AgCl electrode was used as reference and glassy carbon and platinum electrodes were use for standard measurements. Prior to each measurement solutions were fully de-gassed with nitrogen to avoid electrode fouling. Cyclic voltammograms were taken for Tris-borate-EDTA (TBE) buffer, buffer plus cytochrome c, and buffer plus cytochrome c plus two different SS-31 doses as shown in FIG 2 (lower panel). The current (electron diffusion rate) increases almost 200%, as the SS-31 dose is doubled with respect to cytochrome c (cytochrome c:SS-31=1:2). The result indicates that SS-31 promotes the electron diffusion in cytochrome c, making the peptide useful for designing more sensitive bio-detectors.

### Example 4. The peptide D-Arg-2',6'-Dmt-Lys-Phe-NH2 (SS-31) enhances electron capacity in cytochrome c.

Photoluminescence (PL) was carried out to examine the effects of SS-31 on the electronic structure of conduction band of the heme of cytochrome c, an energy state responsible for electronic transport (FIG. 3). A Nd:YD04 laser (532.8 nm) was used to excite electrons in cytochrome c (FIG. 2A). Strong PL emission in cytochrome c state can be clearly identified at 650 nm (FIG. 2B). The PL intensity increased dose-dependently with the addition of SS-31, demonstrating an increase of available electronic states in conduction band in cytochrome c (FIG. 2B). This shows that SS-31 increases electron capacity of conduction band of cytochrome c, concurring with SS-31-mediated increase in current through cytochrome c.

### Example 5. The peptide D-Arg-2',6'-Dmt-Lys-Phe-NH2 (SS-31) induces novel π-π interactions around cytochrome c heme.

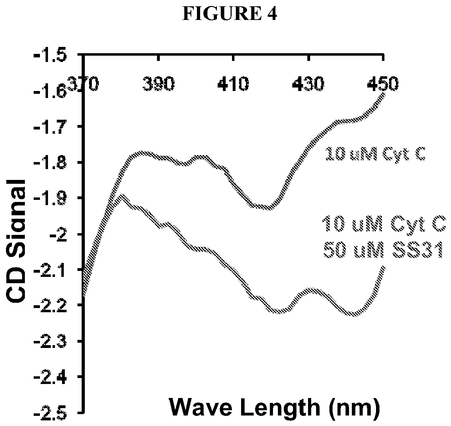

Circular dichroism (Olis spectropolarimeter, DSM20) was carried out to monitor Soret band (negative peak at 415 nm), as a probe for the π-π* heme environment in cytochrome c (FIG. 4). SS-31 promoted a "red" shift of this peak to 440 nm, demonstrating that SS-31 induced a novel heme-tyrosine π-π* transition within cytochrome c, without denaturing (FIG. 4). These results show that SS-31 modifies the immediate environment of the heme, either by providing an additional Tyr for electron tunneling to the heme, or by reducing the distance between endogenous Tyr residues and the heme. The increase in π - *π** interaction around the heme would enhance electron tunneling which would be favorable for electron diffusion.

### Example 6. The peptide D-Arg-2',6'-Dmt-Lys-Phe-NH₂ (SS-31) increases mitochondrial O₂ consumption.

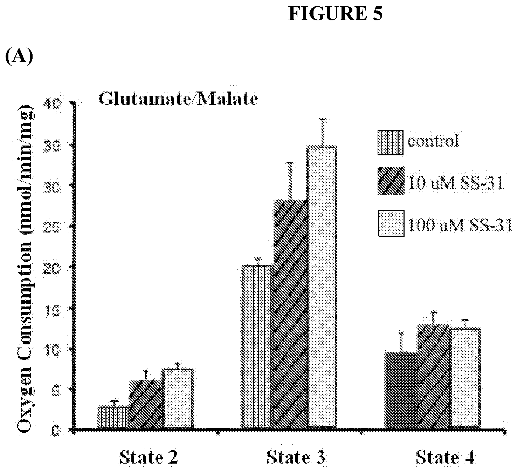

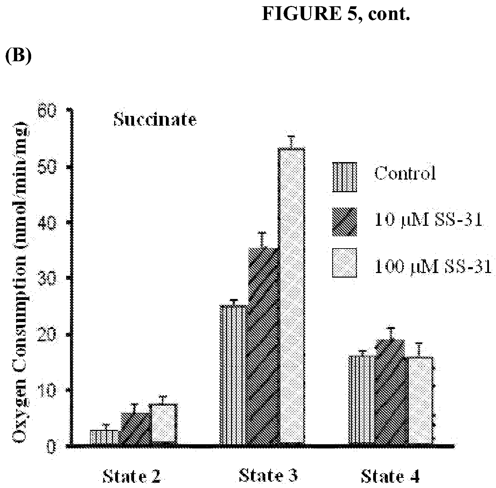

Oxygen consumption of isolated rat kidney mitochondria was determined using the Oxygraph (FIG. 5). Rates of respiration were measured in the presence of different concentrations of SS-31 in state 2 (400 µM ADP only), state 3 (400 µM ADP and 500 µM substrates) and state 4 (substrates only). All experiments were done in triplicate with n = 4-7. The results show that SS-31 promoted electron transfer to oxygen without uncoupling mitochondria (FIG. 5).

### Example 7. The peptide D-Arg-2',6'-Dmt-Lys-Phe-NH2 (SS-31) increases ATP synthesis in isolated mitochondria.

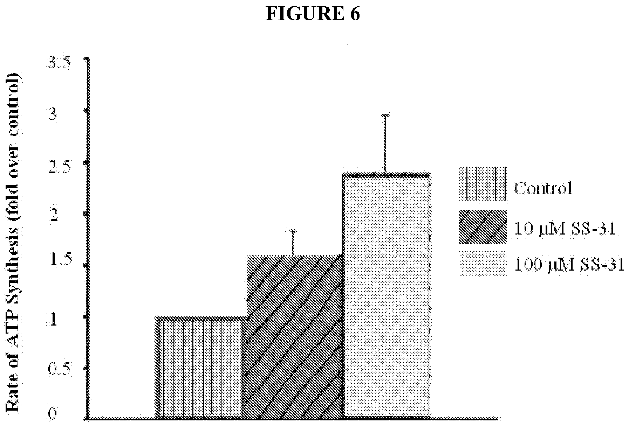

The rate of mitochondrial ATP synthesis was determined by measuring ATP in respiration buffer collected from isolated mitochondria 1 min after addition of 400 mM ADP (FIG. 6). ATP was assayed by HPLC. All experiments were carried out in triplicate, with n=3. Addition of SS-31 to isolated mitochondria dose-dependently increased the rate of ATP synthesis (FIG. 6). These results show that the enhancement of electron transfer by SS-31 is coupled to ATP synthesis.

### Example 8. The peptide D-Arg-2',6'-Dmt-Lys-Phe-NH2 (SS-31) enhances respiration in cytochrome c-depleted mitoplasts.

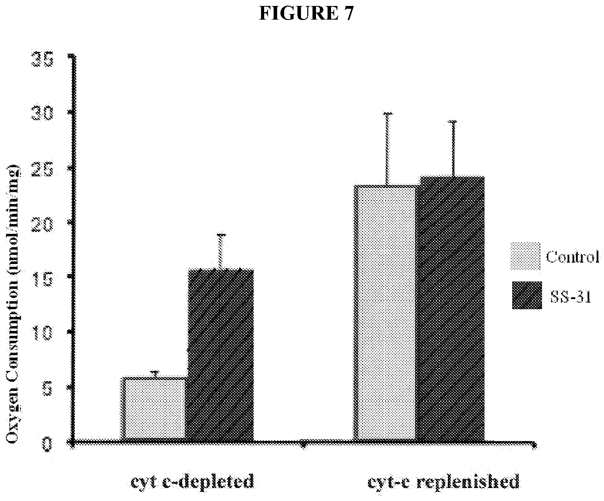

To demonstrate the role of cytochrome c in the action of SS-31 on mitochondrial respiration, the effect of SS-31 on mitochondrial O₂ consumption was determined in cytochrome c-depleted mitoplasts made from once-frozen rat kidney mitochondria (FIG. 7). Rates of respiration were measured in the presence of 500 µM Succinate with or without 100 µM SS-31. The experiment was carried out in triplicate, with n=3. These data show that: 1) SS-31 works via IMM-tightly bound cytochrome c; 2) SS-31 can rescue a decline in functional cytochrome c.

### Example 9. The peptides D-Arg-2',6'-Dmt-Lys-Phe-NH₂ (SS-31) and Phe-D-Arg-Phe-Lys-NH₂ (SS-20) facilitate cytochrome c reduction.

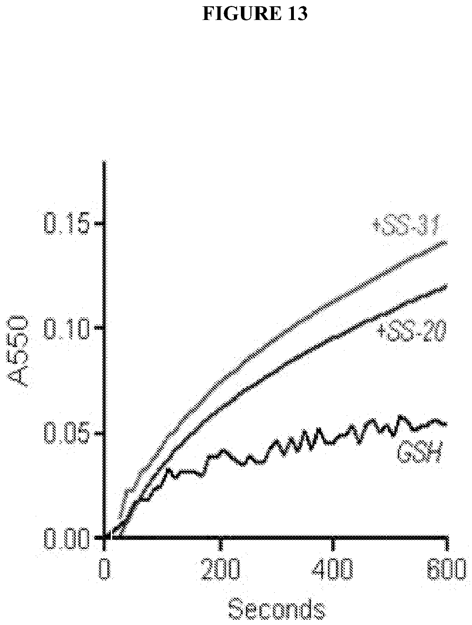

SS-31 and SS-20 can accelerate the kinetics of cytochrome c reduction induced by glutathione (GSH) as a reducing agent (FIG. 13). Reduction of cytochrome c was monitored by increase in absorbance at 550 nm. Addition of GSH resulted in a time-dependent increase in absorbance at 550 nm (FIG. 13). Similar results were obtained using N-acetylcysteine (NAC) as a reducing agent (not shown). The addition of SS-31 alone at 100 µM concentrations did not reduce cytochrome c, but SS-31 dose-dependently increased the rate of NAC-induced cytochrome c reduction, demonstrating that SS-31 does not donate an electron, but can speed up electron transfer.

### Example 10. The peptides D-Are-2\6'-Dmt-Lys-Phe-NH₂ (SS-31) and Phe-D-Arg-Phe-Lys-NH₂ (SS-20) increase mitochondrial electron flux and ATP synthesis.

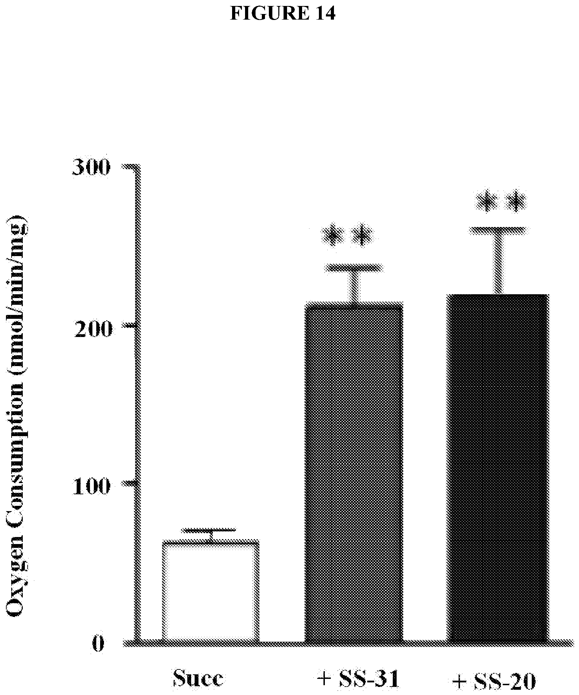

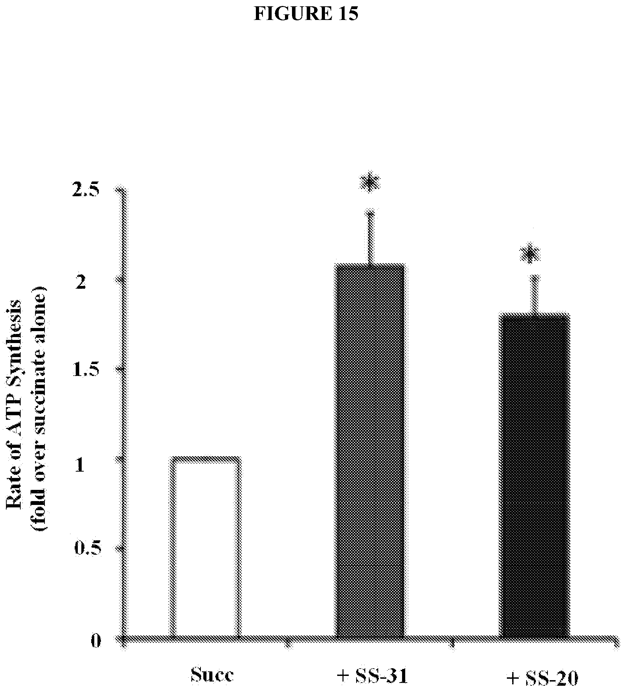

Both SS-20 and SS-31 can promote electron flux, as measured by O₂ consumption in isolated rat kidney mitochondria (FIG. 14). SS-20 or SS-31 was added at 100 µM concentrations to isolated mitochondria in respiration buffer containing 0.5 mM succinate (complex II substrate) and 400 µM ADP. Similar increases in O₂ consumption were observed when low concentrations of complex I substrates (glutamate/malate) were used (data not shown). The increase in electron flux was correlated with a significant increase in the rate of ATP production in isolated mitochondria energized with low concentrations of succinate (FIG. 15). These data show that targeting SS-20 and SS-31 to the IMM can facilitate electron flux in the electron transport chain and improve ATP synthesis, especially under conditions of reduced substrate supply.

### Example 11. Cytochrome c isolation and purification

Methods to isolate and purify cytochrome c are known in the art. One exemplary, non-limiting method is provided. Cytochrome c has several positively charged groups, giving it a pI of around 10. Thus, it is normally bound to the membrane of mitochondria by ionic attraction to the negative charges of the phospholipids on the membrane. The tissue and mitochondria are first broken up by homogenization in a blender at low pH, in an aluminum sulfate solution. The positively charged aluminum ions can displace the cytochrome c from the membrane by binding to the negatively charged phospholipids and free the protein in solution. Excess aluminum sulfate is removed by raising the pH to 8.0, where the aluminum precipitates in the form of aluminum hydroxide.

After filtration to eliminate the precipitated aluminum hydroxide, ion-exchange chromatography is used to separate proteins as a function of their charge. Cytochrome c has several positively charged groups; typically, the column is made out of Amberlite CG-50, a negatively charged or cation-exchange resin.

Once the eluent has been collected, ammonium sulfate precipitation is used to selectively precipitate the remaining contaminant proteins in the cytochrome c preparation. Most proteins precipitate at 80% saturation in ammonium sulfate, whereas cytochrome c remains soluble. The excess of salts present in the solution are then removed by gel filtration chromatography which separates protein on the basis of their size.

To assess the purification, samples of the preparation are collected at each step of the purification. These samples are then assayed for total protein content using the Bradford method, and their cytochrome c concentration is measure by spectrophotometry.

### Example 12: Dissimilatory reduction of soluble sulfates by Desulfovibrio desulfuricans

The bioremediation compositions and methods described herein will be further illustrated by the following example. This example is provided for purposes of illustration only and is not intended to be limiting. The chemicals and other components are presented as typical. Modifications may be derived in view of the foregoing disclosure within the scope of the methods and compositions herein described.

Expression vector construction: Oligonucleotides encoding an aromatic-cationic peptide will be chemically synthesized. The oligonucleotides will be designed to include unique restriction sites at either end that will allow directional cloning into a bacterial plasmid carrying a constitutive promoter upstream of the multiple cloning site. The plasmid will be prepared by restriction digest with enzymes corresponding to the restriction sites on the oligonucleotide ends. The oligonucleotides will be annealed and ligated into the prepared plasmid using conventional techniques of molecular biology. The ligation product will be transformed into E. coli grown on selective media. Several positive clones will be screened for cDNA inserts by DNA sequencing using methods known in the art. Positive clones will be amplified and a stock of the expression construct prepared.

Transformation of *D. desulfuricans*: A 100 ml overnight culture (OD₆₀₀ = 0.6) of *D. desulfuricans* will be centrifuged and the pellet washed three times with sterile water and resuspended in a final volume of 200 µl sterile water. A 30 µl aliqote will be mixed with 4 µl of plasmid preparation (1 µg) and subjected to a 5,000 V/cn electric pulse for 6 ms by an electropulsator apparatus. Recombinant bacteria will be selected on the basis of antibiotic resistance conferred by the recombinant plasmid.

Determination of the sulfate reductase activities of recombinant *D*. *desulfuricans*: wild type and recombinant *D. desufuricans* strains will be tested for the capacity to reduce soluble sulfates. Bacteria will be cultured in a media recommended by the Deutsche Sammlung von Mikroorganismen und Zellkulturen GmbH (German Collection of Microorganisms and Cell Cultures), at 30°C under anaerobic conditions. An aqueous solution of 1280 ppm sulfate will be inoculated with wild-type and recombinant *D*. *desulfuricans* and cultured for 12 hours.

Sulfate measurement: Sulfate concentrations will be measured using a turbidometric technique (Icgen *et al.,* 2006) Sulfate will be precipitated in hydrochloric acid medium with barium chloride to form insoluble barium sulfate crystals. A modified conditioning mixture containing glycerol (104.16 mL), concentrated hydrochloric acid (60.25 mL), and 95% isopropyl alcohol (208.33 mL) will be prepared fresh. For each reaction 2 mL of the cell free supernatant will be diluted 1:50 in Millipore water in a 250 mL conical flask and 5 mL of conditioning mixture added. The entire suspension will be mixed well through stirring. Approximately 1 gm of Barium chloride crystals will be added while stirring is continued for 1 min. The mixture will be allowed to settle for 2 min under static conditions before the turbidity is measured spectrophotometrically at 420 nm. The concentration of sulfate ion will be determined from a curve prepared using standards ranging from 0-40 ppm of Na₂SO₄.

Results: It is predicted that recombinant bacteria expressing aromatic-cationic peptides will display an enhanced rate of dissimilatory sulfate reduction under these conditions.

### EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a nonlimiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

### ASPECTS OF THE INVENTION

1. A method of increasing cytochrome c reduction in a sample containing cytochrome c, comprising contacting the sample with an effective amount of the peptide D-Arg-2',6'-Dmt-Lys-Phe-NH₂.
2. A method of enhancing electron diffusion through cytochrome c in a sample containing cytochrome c, comprising contacting the sample with an effective amount of the peptide D-Arg-2',6'-Dmt-Lys-Phe-NH₂.
3. A method of inducing a *π*-*π* interaction around cytochrome c in a sample containing cytochrome c, comprising contacting the sample with an effective amount of the peptide D-Arg-2',6'-Dmt-Lys-Phe-NH₂.
4. A sensor comprising: cytochrome c doped with a level of the peptide D-Arg-2',6'-Dmt-Lys-Phe-NH₂; and a meter to measure a change in a property of the cytochrome c induced by a change in the level of the peptide D-Arg-2',6'-Dmt-Lys-Phe-NH₂.
5. The sensor of aspect 4 wherein the level of the peptide D-Arg-2',6'-Dmt-Lys-Phe-NH₂ changes in response to variation in at least one of a temperature of the cytochrome c and a pH of the cytochrome c.
6. The sensor of aspect 4 wherein the property is conductivity and the meter includes an anode and a cathode in electrical communication with the cytochrome c.
7. The sensor of aspect 4 wherein the property is photoluminescence and the meter includes a photodetector to measure a change in at least one of an intensity of light emitted by the cytochrome c and wavelength of light emitted by the cytochrome c.
8. A method of sensing comprising measuring a change in a property of cytochrome c doped with a level of the peptide D-Arg-2',6'-Dmt-Lys-Phe-NH₂ induced by a change in the level of the peptide D-Arg-2',6'-Dmt-Lys-Phe-NH₂.
9. The method of aspect 8 wherein the level of the peptide D-Arg-2',6'-Dmt-Lys-Phe-NH₂ changes in response to variation in at least one of a temperature of the cytochrome c and a pH of the cytochrome c.
11. The method of aspect 8 wherein the property is at least one of conductivity, photoluminescent intensity, and photoluminescent wavelength.
12. A switch comprising: cytochrome c; a source of the peptide D-Arg-2*'*,6*'*-Dmt-Lys-Phe-NH₂ in communication with the cytochrome c; and an actuator to control an amount of the peptide D-Arg-2',6'-Dmt-Lys-Phe-NH₂ in communication with the cytochrome c.
13. The switch of aspect 11 wherein the actuator controls at least one of a temperature of the cytochrome c and a pH of the cytochrome c.
14. A method of switching comprising changing a level of the peptide D-Arg-2',6'-Dmt-Lys-Phe-NH₂ in communication with cytochrome c.
15. The method of aspect 13 wherein changing a level of the peptide D-Arg-2',6'-Dmt-Lys-Phe-NH₂ includes varying at least one of a temperature of the cytochrome c and a pH of the cytochrome c.
16. A light-emitting element comprising: cytochrome c doped with an effective amount of the peptide D-Arg-2*'*,6*'*-Dmt-Lys-Phe-NH₂; and a source to stimulate emission of light from the cytochrome c.
17. A method of emitting light, the method comprising stimulating cytochrome c doped with an effective amount of the peptide D-Arg-2',6'-Dmt-Lys-Phe-NH₂.
18. The method of any one of aspects 1-3, wherein the sample comprises a component of a sensor, a conductor, a switch or a light emitting element.
19. A biosensor comprising cytochrome c doped with the peptide D-Arg-2',6'-Dmt-Lys-Phe-NH₂.
20. The biosensor of aspect 19, wherein peptide-doped cytochrome c comprises a mediator in electron flow to an electrode.
21. The biosensor of aspect 19, wherein peptide-doped cytochrome c is immobilized directly on the electrode.
22. The biosensor of aspect 19, wherein the peptide D-Arg-2',6'-Dmt-Lys-Phe-NH₂ and/or cytochrome c are immobilized on a surface within the biosensor.
23. The biosensor of aspect 19, wherein the peptide D-Arg-2',6'-Dmt-Lys-Phe-NH₂ and/or cytochrome c are freely diffusible within the biosensor.
24. A method of detecting a substrate in a sample comprising:
   a) contacting the sample with a biosensor comprising
      i) a redox-active enzyme specific for the substrate
      ii) cytochrome c doped with the peptide D-Arg-2*'*,6*'*-Dmt-Lys-Phe-NH₂; and
      iii) an electrode; and
   b) detecting the flow of electrons within the biosensor.
25. The method of aspect 24, wherein peptide-doped cytochrome c comprises a mediator in electron flow to an electrode.
26. The method of aspect 24, wherein peptide-doped cytochrome c is immobilized directly on the electrode.
27. The method of aspect 24, wherein the peptide D-Arg-2',6'-Dmt-Lys-Phe-NH2 and/or cytochrome c are immobilized on a surface within the biosensor.
28. The method of aspect 24, wherein the peptide D-Arg-2',6'-Dmt-Lys-Phe-NH2 and/or cytochrome c are freely diffusible within the biosensor.
29. A composition for use in the bioremediation of environmental contaminants, comprising: recombinant bacteria expressing one or more aromatic-cationic peptides.
30. The composition of aspect 29, wherein the recombinant bacteria comprise a nucleic acid sequence encoding the one or more aromatic-cationic peptides.
31. The composition of aspect 30, wherein the nucleic acid sequence is expressed under the control of an inducible promoter.
32. The composition of aspect 30, wherein the nucleic acid sequence is expressed under the control of a constitutive promoter.
33. The composition of aspect 30, wherein the nucleic acid sequence comprises a plasmid DNA.
34. The composition of aspect 30, wherein the nucleic acid sequence comprises a genomic insert.
35. The composition of aspect 29, wherein the recombinant bacteria are derived from bacterial species listed in Table 7.
36. A method for bioremediation of environmental contaminants, comprising: contacting a material containing an environmental contaminant with a bioremedial composition comprising recombinant bacteria expressing one or more aromatic-cationic peptides.
37. The method of aspect 36, wherein the environmental contaminant comprises a metal.
38. The method of aspect 37, wherein the metal comprises Sc, Ti, V, Cr, Mn, Fe, Co, Ni, Cu, Zn, Y, Zr, Nb, Mo, Tc, Ru, Pd, Ag, Cd, Hf, Ta, W, Re, Os, Ir, Pt, Au, Hg, Rf, Db, Sg, Bh, Hs, Cn, Al, Ga, In, Sn, Ti, Pb, or Bi.
39. The method of aspect 36, wherein the environmental contaminant comprises a non-metal.
40. The method of aspect 39, wherein the non-metal comprises sulfate.
41. The method of aspect 36, wherein the environmental contaminant comprises a perchlorate.
42. The method of aspect 41, wherein the perchlorate comprises NH₄ClO₄, CsClO₄, LiClO₄, Mg(ClO₄)₂, HClO₄, KClO₄, RbClO₄, AgClO₄, or NaClO₄.
43. The method of aspect 36, wherein the environmental contaminant comprises a nitrate.
44. The method of aspect 43, wherein the nitrate comprises HNO₃, LiNO₃, NaNO₃, KNO₃, RbNO₃, CsNO₃, Be(NO₃)₂, Mg(NO₃)₂, Ca(NO₃)₂, Sr(NO₃)₂, Ba(NO₃)₂, Sc(NO₃)₃, Cr(NO₃)₃, Mn(NO₃)₂, Fe(NO₃)₃, Co(NO₃)₂, Ni(NO₃)₂, Cu(NO₃)₂, Zn(NO₃)₂, Pd(NO₃)₂, Cd(NO₃)₂, Hg(NO₃)₂, Pb(NO₃)₂, or Al(NO₃)₃.
45. The method of aspect 36, wherein the environmental contaminant comprises a radionuclide.
46. The method of aspect 45, wherein the radionuclide comprises an actinide.
47. The method of aspect 45, wherein the radionuclide comprises uranium.
48. The method of aspect 36, wherein the environmental contaminant comprises methyl-tert-butyl-ether (MTBE), vinyl chloride, or dichloroethylene.
49. The method of aspect 36, wherein bioremediation is performed *in situ.*
50. The method of aspect 36, wherein bioremediation is performed *ex situ.*
51. The method of aspect 36, wherein the bacteria comprise a nucleic acid sequence encoding the one or more aromatic-cationic peptides.
52. The method of aspect 51, wherein the nucleic acid sequence is expressed under the control of an inducible promoter.
53. The method of aspect 51, wherein the nucleic acid sequence is expressed under the control of a constitutive promoter.
54. The method of aspect 51, wherein the nucleic acid sequence comprises a plasmid DNA.
55. The method of aspect 51, wherein the nucleic acid sequence comprises a genomic insert
56. The method of aspect 36, wherein the recombinant bacteria are derived from bacterial species listed in Table 7.
57. The method of any one of aspects 36-56, wherein the aromatic-cationic peptide comprises D-Arg-2',6'-Dmt-Lys-Phe-NH₂.

## Claims

1. A composition for use in the bioremediation of environmental contaminants, comprising recombinant bacteria expressing one or more aromatic-cationic peptides, wherein the peptide is selected from the group consisting of:
D-Arg-2*'*,6*'*-Dmt-Lys-Phe-NH₂;
Tyr-D-Arg-Phe-Lys-NH₂;
2',6'-Dmt-D-Arg-Phe-Lys-NH_{2;}
Phe-D-Arg-Phe-Lys-NH_{2;}
D-Arg-2',6'-Dmt-Lys-Trp-NH₂;
D-Arg-Trp-Lys-Trp-NH₂;
D-Arg-2',6'-Dmt-Lys-Phe-Met-NH₂;
H-D-Arg-2',6'-Dmt-Lys(*N^{α}*Me)-Phe-NH₂;
H-D-Arg-2*'*,6*'*-Dmt-Lys-Phe(*N*Me)-NH₂;
H-D-Arg-2',6'-Dmt-Lys(*N^{α}*Me)-Phe(*N*Me)-NH₂;
H-D-Arg(*N*^{α}Me)-2',6'-Dmt(*N*Me)-Lys(*N*^{α}Me)-Phe(*N*Me)-NH₂;
D-Arg-2',6'-Dmt-Lys-Phe-Lys-Trp-NH₂;
D-Arg-2*'*,6*'*-Dmt-Lys-2*'*,6*'*-Dmt-Lys-Trp-NH₂;
D-Arg-2',6'-Dmt-Lys-Phe-Lys-Met-NH₂;
D-Arg-2*'*,6*'*-Dmt-Lys-2*'*,6*'*-Dmt-Lys-Met-NH₂;
H-D-Arg-2*'*,6*'*-Dmt-Lys-Phe-Sar-Gly-Cys-NH₂;
H-D-Arg-Ψ[CH₂-NH]-2*'*,6*'*-Dmt-Lys-Phe-NH₂;
H-D-Arg-2*'*,6*'*-Dmt-Ψ[CH₂-NH]Lys-Phe-NH₂;
H-D-Arg-2',6'-Dmt-LysΨ[CH₂-NH]Phe-NH₂;
H-D-Arg-2*'*,6*'*-Dmt-Ψ[CH₂-NH]Lys-Ψ[CH₂-NH]Phe-NH₂;
Lys-D-Arg-Tyr-NH₂;
Tyr-D-Arg-Phe-Lys-NH₂;
2',6'-Dmt-D-Arg-Phe-Lys-NH_{2;}
Phe-D-Arg-Phe-Lys-NH₂;
Phe-D-Arg-2',6'-Dmt-Lys-NH₂;
D-Arg-2*'*,6*'*-Dmt-Lys-Phe-NH₂;
H-Phe-D-Arg-Phe-Lys-Cys-NH₂;
Lys-D-Arg-Tyr-NH₂;
D-Tyr-Trp-Lys-NH₂;
Trp-D-Lys-Tyr-Arg-NH₂;
Tyr-His-D-Gly-Met;
Tyr-D-Arg-Phe-Lys-Glu-NH₂ ;
Met-Tyr-D-Lys-Phe-Arg;
D-His-Glu-Lys-Tyr-D-Phe-Arg;
Lys-D-Gln-Tyr-Arg-D-Phe-Trp-NH₂;
Phe-D-Arg-Lys-Trp-Tyr-D-Arg-His;
Gly-D-Phe-Lys-Tyr-His-D-Arg-Tyr-NH₂;
Val-D-Lys-His-Tyr-D-Phe-Ser-Tyr-Arg-NH₂;
Trp-Lys-Phe-D-Asp-Arg-Tyr-D-His-Lys;
Lys-Trp-D-Tyr-Arg-Asn-Phe-Tyr-D-His-NH₂;
Thr-Gly-Tyr-Arg-D-His-Phe-Trp-D-His-Lys;
Asp-D-Trp-Lys-Tyr-D-His-Phe-Arg-D-Gly-Lys-NH₂;
D-His-Lys-Tyr-D-Phe-Glu-D-Asp-D-His-D-Lys-Arg-Trp-NH₂;
Ala-D-Phe-D-Arg-Tyr-Lys-D-Trp-His-D-Tyr-Gly-Phe;
Tyr-D-His-Phe-D-Arg-Asp-Lys-D-Arg-His-Trp-D-His-Phe;
Phe-Phe-D-Tyr-Arg-Glu-Asp-D-Lys-Arg-D-Arg-His-Phe-NH₂;
Phe-Tyr-Lys-D-Arg-Trp-His-D-Lys-D-Lys-Glu-Arg-D-Tyr-Thr;
Tyr-Asp-D-Lys-Tyr-Phe-D-Lys-D-Arg-Phe-Pro-D-Tyr-His-Lys;
Glu-Arg-D-Lys-Tyr-D-Val-Phe-D-His-Trp-Arg-D-Gly-Tyr-Arg-D-Met-NH₂;
Arg-D-Leu-D-Tyr-Phe-Lys-Glu-D-Lys-Arg-D-Trp-Lys-D-Phe-Tyr-D-Arg-Gly;
D-Glu-Asp-Lys-D-Arg-D-His-Phe-Phe-D-Val-Tyr-Arg-Tyr-D-Tyr-Arg-His-Phe-NH₂;
Asp-Arg-D-Phe-Cys-Phe-D-Arg-D-Lys-Tyr-Arg-D-Tyr-Trp-D-His-Tyr-D-Phe-Lys-Phe;
His-Tyr-D-Arg-Trp-Lys-Phe-D-Asp-Ala-Arg-Cys-D-Tyr-His-Phe-D-Lys-Tyr-His-Ser-NH₂;
Gly-Ala-Lys-Phe-D-Lys-Glu-Arg-Tyr-His-D-Arg-D-Arg-Asp-Tyr-Trp-D-His-Trp-His-D-Lys-Asp; and
Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂.

2. The composition of claim 1, wherein the recombinant bacteria comprise a nucleic acid sequence encoding the one or more aromatic-cationic peptides.

3. The composition of claim 2, wherein the nucleic acid sequence is expressed under the control of an inducible promoter or a constitutive promoter.

4. The composition of claim 2, wherein the nucleic acid sequence comprises a plasmid DNA.

5. The composition of claim 2, wherein the nucleic acid sequence comprises a genomic insert.

6. The composition of claim 1, wherein the recombinant bacteria are derived from bacterial species listed in Table 7.

7. Use of the composition as defined in any of the preceding claims for bioremediation of environmental contaminants.

8. A method for bioremediation of environmental contaminants, comprising contacting a material containing an environmental contaminant with a bioremedial composition, the composition comprising recombinant bacteria expressing one or more aromatic-cationic peptides, wherein the peptide is selected from the group consisting of:
D-Arg-2*'*,6*'*-Dmt-Lys-Phe-NH₂;
Tyr-D-Arg-Phe-Lys-NH₂;
2',6'-Dmt-D-Arg-Phe-Lys-NH_{2;}
Phe-D-Arg-Phe-Lys-NH₂;
D-Arg-2',6'-Dmt-Lys-Trp-NH₂;
D-Arg-Trp-Lys-Trp-NH₂;
D-Arg-2',6'-Dmt-Lys-Phe-Met-NH₂;
H-D-Arg-2',6'-Dmt-Lys(*N^{α}*Me)-Phe-NH₂;
H-D-Arg-2',6'-Dmt-Lys-Phe(*N*Me)-NH₂;
H-D-Arg-2',6'-Dmt-Lys(*N^{α}*Me)-Phe(*N*Me)-NH₂;
H-D-Arg(*N^{α}*Me)-2',6'-Dmt(*N*Me)-Lys(*N^{α}*Me)-Phe(*N*Me)-NH₂;
D-Arg-2*'*,6*'*-Dmt-Lys-Phe-Lys-Trp-NH₂;
D-Arg-2',6'-Dmt-Lys-2',6'-Dmt-Lys-Trp-NH₂;
D-Arg-2*'*,6'-Dmt-Lys-Phe-Lys-Met-NH₂;
D-Arg-2',6'-Dmt-Lys-2*'*,6*'*-Dmt-Lys-Met-NH₂;
H-D-Arg-2*'*,6*'*-Dmt-Lys-Phe-Sar-Gly-Cys-NH₂;
H-D-Arg-Ψ[CH₂-NH]-2*'*,6*'*-Dmt-Lys-Phe-NH₂;
H-D-Arg-2*'*,6*'*-Dmt-Ψ[CH₂-NH]Lys-Phe-NH₂;
H-D-Arg-2*'*,6*'*-Dmt-LysΨ[CH₂-NH]Phe-NH₂;
H-D-Arg-2*'*,6*'*-Dmt-Ψ[CH₂-NH]Lys-Ψ[CH₂-NH]Phe-NH₂;
Lys-D-Arg-Tyr-NH₂;
Tyr-D-Arg-Phe-Lys-NH₂;
2',6'-Dmt-D-Arg-Phe-Lys-NH_{2;}
Phe-D-Arg-Phe-Lys-NH₂;
Phe-D-Arg-2',6'-Dmt-Lys-NH₂;
D-Arg-2*'*,6*'*-Dmt-Lys-Phe-NH₂;
H-Phe-D-Arg-Phe-Lys-Cys-NH₂;
Lys-D-Arg-Tyr-NH₂;
D-Tyr-Trp-Lys-NH₂;
Trp-D-Lys-Tyr-Arg-NH₂;
Tyr-His-D-Gly-Met;
Tyr-D-Arg-Phe-Lys-Glu-NH₂ ;
Met-Tyr-D-Lys-Phe-Arg;
D-His-Glu-Lys-Tyr-D-Phe-Arg;
Lys-D-Gln-Tyr-Arg-D-Phe-Trp-NH₂;
Phe-D-Arg-Lys-Trp-Tyr-D-Arg-His;
Gly-D-Phe-Lys-Tyr-His-D-Arg-Tyr-NH₂;
Val-D-Lys-His-Tyr-D-Phe-Ser-Tyr-Arg-NH₂;
Trp-Lys-Phe-D-Asp-Arg-Tyr-D-His-Lys;
Lys-Trp-D-Tyr-Arg-Asn-Phe-Tyr-D-His-NH₂;
Thr-Gly-Tyr-Arg-D-His-Phe-Trp-D-His-Lys;
Asp-D-Trp-Lys-Tyr-D-His-Phe-Arg-D-Gly-Lys-NH₂;
D-His-Lys-Tyr-D-Phe-Glu-D-Asp-D-His-D-Lys-Arg-Trp-NH₂;
Ala-D-Phe-D-Arg-Tyr-Lys-D-Trp-His-D-Tyr-Gly-Phe;
Tyr-D-His-Phe-D-Arg-Asp-Lys-D-Arg-His-Trp-D-His-Phe;
Phe-Phe-D-Tyr-Arg-Glu-Asp-D-Lys-Arg-D-Arg-His-Phe-NH2;
Phe-Tyr-Lys-D-Arg-Trp-His-D-Lys-D-Lys-Glu-Arg-D-Tyr-Thr;
Tyr-Asp-D-Lys-Tyr-Phe-D-Lys-D-Arg-Phe-Pro-D-Tyr-His-Lys;
Glu-Arg-D-Lys-Tyr-D-Val-Phe-D-His-Trp-Arg-D-Gly-Tyr-Arg-D-Met-NH₂;
Arg-D-Leu-D-Tyr-Phe-Lys-Glu-D-Lys-Arg-D-Trp-Lys-D-Phe-Tyr-D-Arg-Gly;
D-Glu-Asp-Lys-D-Arg-D-His-Phe-Phe-D-Val-Tyr-Arg-Tyr-D-Tyr-Arg-His-Phe-NH₂;
Asp-Arg-D-Phe-Cys-Phe-D-Arg-D-Lys-Tyr-Arg-D-Tyr-Trp-D-His-Tyr-D-Phe-Lys-Phe;
His-Tyr-D-Arg-Trp-Lys-Phe-D-Asp-Ala-Arg-Cys-D-Tyr-His-Phe-D-Lys-Tyr-His-Ser-NH₂;
Gly-Ala-Lys-Phe-D-Lys-Glu-Arg-Tyr-His-D-Arg-D-Arg-Asp-Tyr-Trp-D-His-Trp-His-D-Lys-Asp; and
Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂.

9. The method of claim 8, wherein the environmental contaminant comprises a metal, wherein the metal comprises one or more of Sc, Ti, V, Cr, Mn, Fe, Co, Ni, Cu, Zn, Y, Zr, Nb, Mo, Tc, Ru, Pd, Ag, Cd, Hf, Ta, W, Re, Os, Ir, Pt, Au, Hg, Rf, Db, Sg, Bh, Hs, Cn, Al, Ga, In, Sn, Ti, Pb, or Bi.

10. The method of claim 8, wherein the environmental contaminant comprises a non-metal, wherein the non-metal comprises one or more of sulfate or perchlorate, wherein the perchlorate comprises one or more of NH₄CIO₄, CsClO₄, LiClO₄, Mg(ClO₄)₂, HClO₄, KClO₄, RbClO₄, AgClO₄, or NaClO₄.

11. The method of claim 8, wherein the environmental contaminant comprises a nitrate, wherein the nitrate comprises one or more of HNO₃, LiNO₃, NaNO₃, KNO₃, RbNO₃, CsNO₃, Be(NO₃)₂, Mg(NO₃)₂, Ca(NO₃)₂, Sr(NO₃)₂, Ba(NO₃)₂, Sc(NO₃)₃, Cr(NO₃)₃, Mn(NO₃)₂, Fe(NO₃)₃, Co(NO₃)₂, Ni(NO₃)₂, Cu(NO₃)₂, Zn(NO₃)₂, Pd(NO₃)₂, Cd(NO₃)₂, Hg(NO₃)₂, Pb(NO₃)₂, or Al(NO₃)₃.

12. The method of claim 8, wherein the environmental contaminant comprises a radionuclide, wherein the radionuclide comprises one or more of an actinide or uranium.

13. The method of claim 8, wherein the environmental contaminant comprises one or more of methyl-tert-butyl-ether (MTBE), vinyl chloride, or dichloroethylene.

14. The method of claim 8, wherein bioremediation is performed *in situ* or *ex situ.*

15. The method of claim 8, wherein the recombinant bacteria are derived from bacterial species listed in Table 7.
